# EUROPEAN PATENT SPECIFICATION

(11) **EP 2 638 394 B1**
(45) Date of publication and mention of the grant of the patent: **10.05.2017**
(21) Application number: 11839316.4
(22) Date of filing: 14.11.2011
(51) Int. Cl.: G01N 33/53, C07K 16/18, C07K 16/30, G01N 33/574

(54) **HISTONE PROTEIN UBIQUITINATION AS A CANCER BIOMARKER**
HISTONPROTEINUBIQUITINIERUNG ALS KREBSBIOMARKER
UBIQUITINATION DE LA PROTÉINE D'HISTONE UTILISÉE COMME BIOMARQUEUR DU CANCER

(30) Priority: 12.11.2010 US 412905 P; 12.11.2010 AU 2010241416
(43) Date of publication of application: 18.09.2013
(73) Proprietor: Northern Sydney Local Health District, New South Wales 2065 (AU)
(72) Inventor: MARSH, Deborah Joy, Naremburn NSW 2065 (AU); HAHN, Michael Anthony, Frenchs Forest NSW 2086 (AU)
(74) Representative: Cabinet Plasseraud
(86) International application number: PCT/AU2011/001468
(87) International publication number: WO 2012/061904

(56) References cited:
- KR-A- 20020 086 987
- KR-A- 20050 073 430
- KR-A- 20090 092 495
- VIKKI M WEAKE ET JERRY L. WORKMAN: "Histone ubiquitination: Triggering gene activity", MOLECULAR CELL, vol. 29, 28 March 2008 (2008-03-28), pages 653-663, XP002722043,
- Ping Zhu ET AL: "A Histone H2A Deubiquitinase Complex Coordinating Histone Acetylation and H1 Dissociation in Transcriptional Regulation", MOLECULAR CELL., vol. 27, no. 4, 1 August 2007 (2007-08-01) , pages 609-621, XP055235193, US ISSN: 1097-2765, DOI: 10.1016/j.molcel.2007.07.024

## Description

### TECHNICAL FIELD

The present invention relates generally to the field of cancer diagnosis. More specifically, the present invention relates to the identification and use of biomarkers for the diagnosis and prognosis of cancer.

### BACKGROUND

Cancer is a group of diseases characterized by the uncontrolled growth and spread of abnormal cells, and is responsible for a large number of annual deaths in both developed and undeveloped countries. It is estimated that more than 1.5 million new cases of cancer will be diagnosed in 2010 in the US alone with direct medical costs estimated at US$102.8 billion (American Cancer Society "Cancer Facts and Figures 2010", Atlanta: American Cancer Society; 2010).

The early diagnosis of cancer is of primary importance as it can facilitate more effective treatment of the disease. However, many types of cancer are difficult to detect until the disease is significantly advanced. This arises at least in part from the inadequacies of current diagnostic tests many of which are based on unreliable indicators. For example, the diagnosis of parathyroid cancer generally relies on the detection of increased levels of calcium and/or parathyroid hormone in the blood, but these tests do not discriminate between benign or malignant disease. Clear histological criteria are not available. Beyond these tests there are no biochemical or radiological assays that can definitively diagnose parathyroid cancer, and physical symptoms can easily be confused with those of benign hyperparathyroidism due to hyperplasia or adenomas. A clear diagnosis of parathyroid carcinoma is frequently only conclusively made upon recurrence of the tumour.

Similarly, diagnostic tests for many other types of cancer can be unreliable and are known to produce false positive and/or false negative results in some cases.

Increasingly, the importance of a 'molecular' diagnosis, rather than purely a histological one that only addresses characteristics of a tumour such as those noted in TNM classifications, is being recognised. Molecular diagnoses using newly discovered biomarkers in a range of tumour types will increasingly play a part in prognostics for cancer patients, as well as drive decisions regarding which molecular therapies a patient is most likely to respond to. Molecular markers of genetic mutations will be of particular importance in this area. For example, a marker of "BRCAness" that could easily identify tumours (breast, ovarian, pancreatic) that harbour *BRCA* (breast cancer, early onset) mutations would be of major value given the need to rapidly identify these tumours and implement targeted therapies (e.g. PARP inhibitors).

A need exists for new methods of diagnosing cancer and in particular molecular-based methods of diagnosing cancer.

### SUMMMARY OF THE INVENTION

The ubiquitination of histone proteins including histone proteins 2A and 2B (H2A and H2B) is considered to be a marker of active chromatin and thus increased gene expression. Therefore, it has been postulated that the ubiquitination of histone proteins could be increased in proliferating cancer cells. Unexpectedly, the present inventors have identified that ubiquitination of histone proteins is decreased in cancer cells.

Accordingly, in a first aspect the invention provides a method for diagnosing cancer in a subject, the method comprising measuring histone protein monoubiquitination in a first cell of the subject, wherein decreased histone protein monoubiquitination in said first cell compared to that of a second non-cancerous cell is diagnostic of cancer.

In one embodiment of the first aspect, the histone protein is a histone H2B protein.

In a second aspect, the invention provides a method for diagnosing cancer in a subject, the method comprising measuring histone 2B protein monoubiquitination in a first cell of the subject, wherein decreased histone 2B protein monoubiquitination in said first cell compared to that of a second non-cancerous cell is diagnostic of cancer.

In one embodiment of the first and second aspects, the method is an *in vitro* or an *ex vivo* method.

In one embodiment of the first and second aspects, the method comprises measuring histone 2B protein monoubiquitination in a first cell obtained from the subject.

In one embodiment of the first and second aspects, the method comprises measuring histone 2B protein monoubiquitination in a second cell obtained from the subject.

In one embodiment of the first and second aspects, the method comprises measuring histone 2B protein monoubiquitination in a second cell obtained from a different individual.

In one embodiment of the first and second aspects, the second non-cancerous cell is from the same subject.

In one embodiment of the first and second aspects, the second non-cancerous cell is from a different individual.

In one embodiment of the first and second aspects, the first and second cells are the same cell type.

In one embodiment of the first and second aspects, the first and second cells are different cell types.

In one embodiment of the first and second aspects, the monoubiquitination is at lysine residue 121 of a human histone H2B protein of 126 amino acids in length.

In one embodiment of the first and second aspects, the monoubiquitination is at a lysine residue in a shortened human histone H2B protein, said lysine residue corresponding to lysine residue 121 of a human histone H2B protein of 126 amino acids in length.

In one embodiment of the first and second aspects, either or both of the first and second cells are in a biological sample.

In one embodiment of the first and second aspects, the biological sample is a biopsy.

In one embodiment of the first and second aspects, the cancer is parathyroid cancer, and/or cancer of the colon, breast, lung, prostate, ovary, brain, and/or skin.

In one embodiment of the first and second aspects, the cancer is cancer of the pancreas, liver, oesophagus, thyroid gland, endometrium, pituitary gland, adrenal gland, breast, and/or prostate gland.

In one embodiment of the first and second aspects, the cancer is parathyroid cancer, breast cancer, colorectal cancer, lung cancer, melanom, adrenal cancer, and/or ovarian cancer.

In one embodiment of the first and second aspects, the cancer is characterised by one or more genetic mutations in the *BRCA1* (breast cancer 1, early onset) gene.

In one embodiment of the first and second aspects, the cancer is characterised by one or more genetic mutations in the *BRCA2* (breast cancer 2, early onset) gene.

In one embodiment of the first and second aspects, the cancer is characterised by one or more genetic mutations in the *BRCA1* gene and one or more genetic mutations in the *BRCA2* gene.

In one embodiment of the first and second aspects, the cancer is a breast cancer tumour, the tumour comprising cells comprising one or more genetic mutations in the *BRCA1* gene.

In one embodiment of the first and second aspects, the cancer is a breast cancer tumour, the tumour comprising cells comprising one or more genetic mutations in the *BRCA2* gene.

In one embodiment of the first and second aspects, the cancer is a breast cancer tumour, the tumour comprising cells comprising one or more genetic mutations in the *BRCA1* gene and one or more mutations in the *BRCA2* gene. In one embodiment of the first and second aspects, the cancer is parathyroid cancer.

In one embodiment of the first and second aspects, the histone protein monoubiquitination in said first cell is decreased by at least 10% compared to histone protein monoubiquitination in said second non-cancerous cell.

In one embodiment of the first and second aspects, the measuring of histone protein monoubiquitination is performed using an antibody.

In one embodiment of the first and second aspects, the measuring of histone protein monoubiquitination is performed using any one or more of immunohistochemical staining, Western blotting and fluorescent activated cell sorting.

In a third aspect, is disclosed a kit for the diagnosis of cancer, the kit comprising a reagent that specifically binds to a monoubiquitinated histone protein.

In a fourth aspect, is disclosed a kit when used for the diagnosis of cancer according to the method of the first or second aspect, the kit comprising a reagent that specifically binds to a monoubiquitinated histone protein.

In a fifth aspect, is disclosed a kit for the diagnosis of cancer, the kit comprising a reagent that specifically binds to a monoubiquitinated histone protein.

In a sixth aspect, is disclosed the use of a kit for the diagnosis of cancer according to the method of the first or second aspect, the kit comprising a reagent that specifically binds to a monoubiquitinated histone protein.

In one embodiment of the third, fourth, fifth and sixth aspects, the histone protein is a human histone H2B protein of 126 amino acids in length that is monoubiquitinated at lysine residue 121.

In one embodiment of the third, fourth, fifth and sixth aspects, the histone protein is a human histone H2B protein shorter than 126 amino acids in length that is monoubiquitinated at a lysine residue corresponding to lysine residue 121 of a human histone H2B protein of 126 amino acids in length.

In one embodiment of the third, fourth, fifth and sixth aspects, the reagent is an antibody capable of specifically binding to the monoubiquitinated lysine at residue 121.

In one embodiment of the third, fourth, fifth and sixth aspects, the cancer is parathyroid cancer and/or cancer of the colon, breast, lung, prostate, ovary, brain, and/or skin.

In one embodiment of the third, fourth, fifth and sixth aspects, the cancer is cancer of the pancreas, liver, oesophagus, thyroid gland, endometrium, pituitary gland, adrenal gland, breast, and/or prostate gland.

In one embodiment of the third, fourth, fifth and sixth aspects, the cancer is parathyroid cancer, breast cancer, colorectal cancer, lung cancer, melanoma, adrenal cancer, and/or ovarian cancer.

In one embodiment of the third, fourth, fifth and sixth aspects, the cancer is characterised by a mutation in the *BRCA1* gene.

In one embodiment of the third, fourth, fifth and sixth aspects, the cancer is parathyroid cancer.

In a seventh aspect is disclosed a method of screening for an agent that modulates monoubiquitination of a histone protein, the method comprising determining whether a candidate agent reduces or augments binding between CDC73 and RNF20.

In one embodiment of this aspect, the determining comprises:
(i) administering the candidate agent to a first cell population comprising CDC73 and RNF20;
(ii) analysing binding interactions between CDC73 and RNF20 in the first cell population; and
(iii) comparing binding interactions analysed in (ii) with CDC73 and RNF20 binding interactions analysed in a second cell population to which the candidate agent has not been administered.

In one embodiment of the seventh aspect, the histone protein is a histone H2B protein.

In one embodiment of the seventh aspect, the monoubiquitination is at lysine residue 121 of a human histone H2B protein of 126 amino acids in length.

In one embodiment of the seventh aspect, the monoubiquitination is at a lysine residue in a shortened human histone H2B protein, said lysine residue corresponding to lysine residue 121 of a human histone H2B protein of 126 amino acids in length.

In an eighth aspect is disclosed a method of screening for an agent that reduces or augments binding between CDC73 and RNF20, the method comprising:
(i) administering a candidate agent to a first cell population comprising CDC73 and RNF20;
(ii) analysing binding interactions between CDC73 and RNF20 in the first cell population; and
(iii) comparing binding interactions analysed in (ii) with CDC73 and RNF20 binding interactions analysed in a second cell population to which the candidate agent has not been administered.

### BRIEF DESCRIPTION OF THE FIGURES

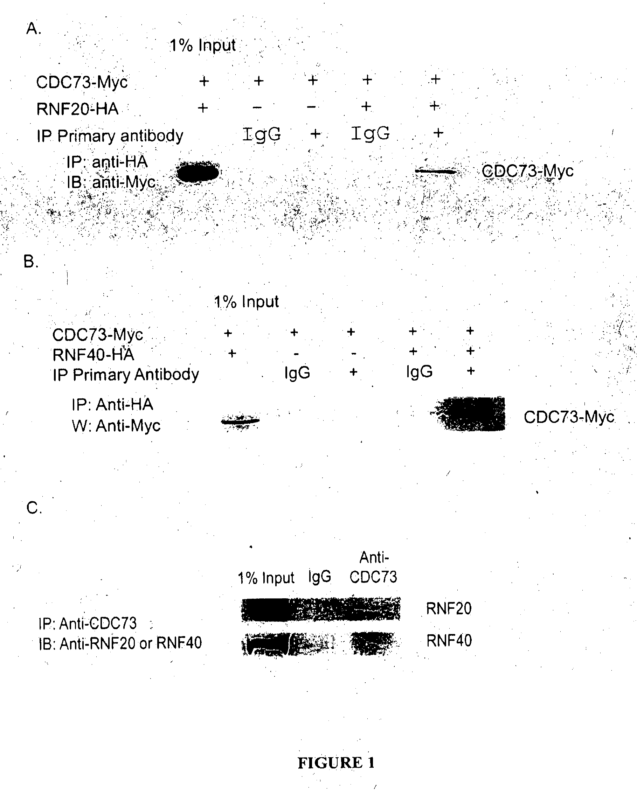

**Figure 1** demonstrates that the ring finger proteins RNF20 and RNF40 are parafibromin (CDC73) interacting proteins. To validate the parafibromin/RNF20 and parafibromin/RNF40 interactions identified in a yeast two-hybrid assay co-immunoprecipitation of overexpressed (A and B) or endogenous (C) RNF20 or RNF40 and parafibromin in HEK293 cells was performed. (IP - immunoprecipitation, IB - immunoblot).

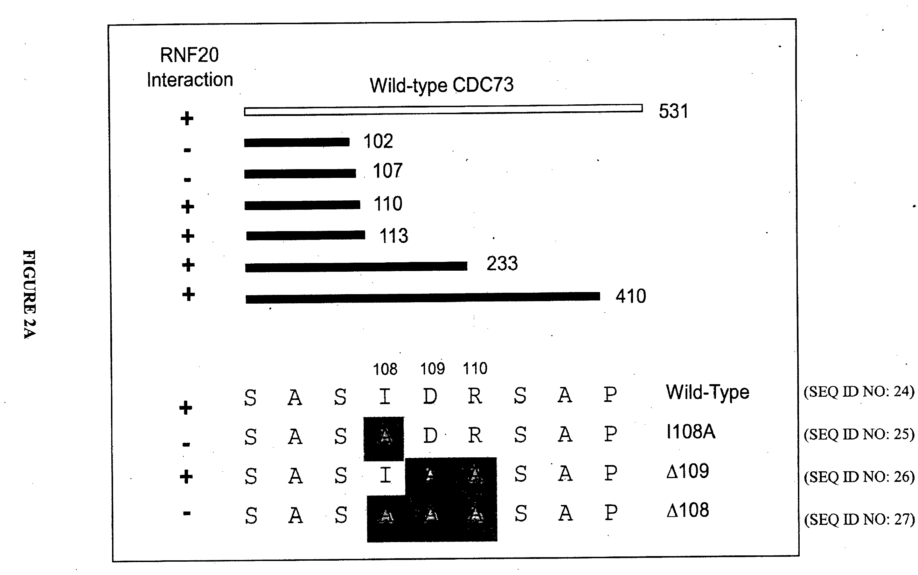

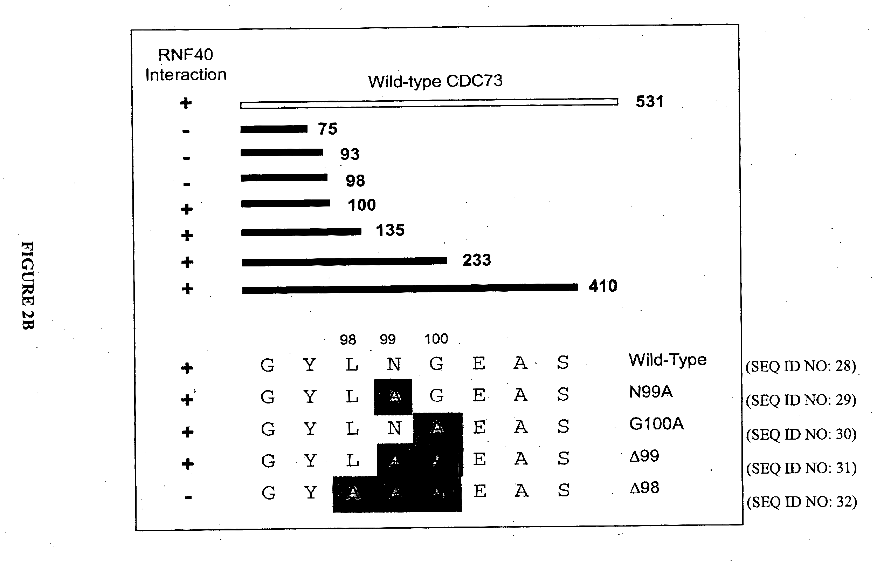

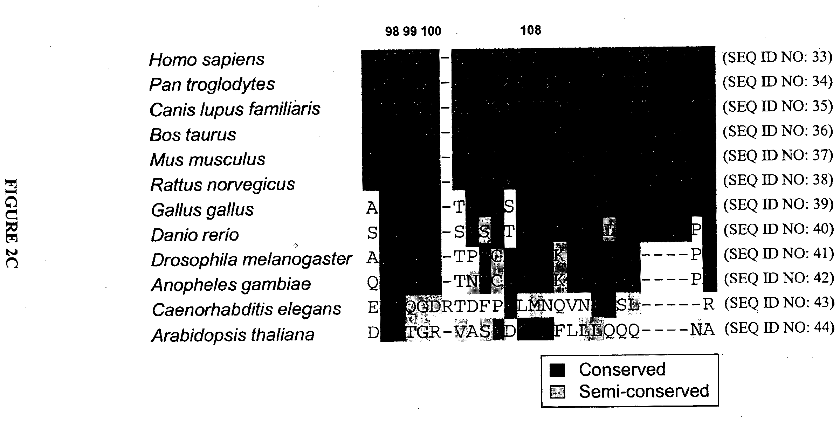

**Figure 2** shows that residue 108 of parafibromin (CDC73) is required for interaction with RNF20. A number of parafibromin truncation mutants were co-transformed into yeast cells and their ability to interact with RNF20 assessed using the Y2H assay (A - Top panel). Residues 108-110 in full length parafibromin were then mutated individually or in combination to alanine and used in the Y2H assay to identify the residues required for RNF20 binding (A - Bottom panel). The parafibromin truncation mutants were also assessed for their ability to interact with RNF40 using the Y2H assay (B - Top panel). Residues 98-100 of parafibromin were mutated individually or in combination to alanine and used in the Y2H assay to identify the residues required for RNF40 binding (B - Bottom panel). A multiple species sequence alignment of the parafibromin protein sequence within the region containing residue 108 is shown in (C). Conserved amino acids are shaded dark grey while semi-conserved amino acids are shaded light grey (C).

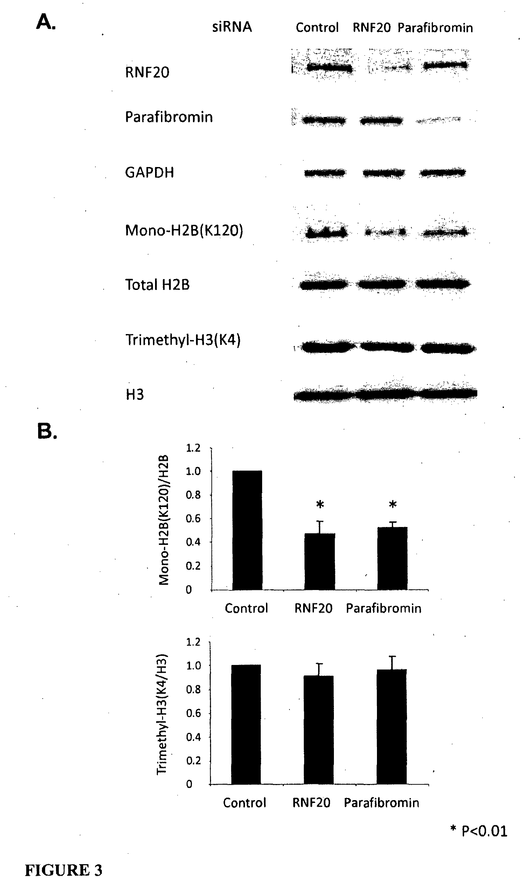

**Figure 3** demonstrates that parafibromin (CDC73) regulates H2B monoubiquitination at K120. The expression of RNF20 (Lane 2) or parafibromin (Lane 3) was reduced by siRNA knockdown in HEK293 cells and the levels of monoubiquitinated H2B (K120), total H2B, trimethylated H3 (K4) and total H3 analysed by western blot (A). The mean levels (+/- SEM) of monoubiquitinated H2B (K120) relative to total H2B and trimethylated H3 (K4) relative to total H3 from three separate experiments were determined in the presence of RNF20 or parafibromin knockdown and are shown. All treatments were normalised to control. (B). (*P<0.01)

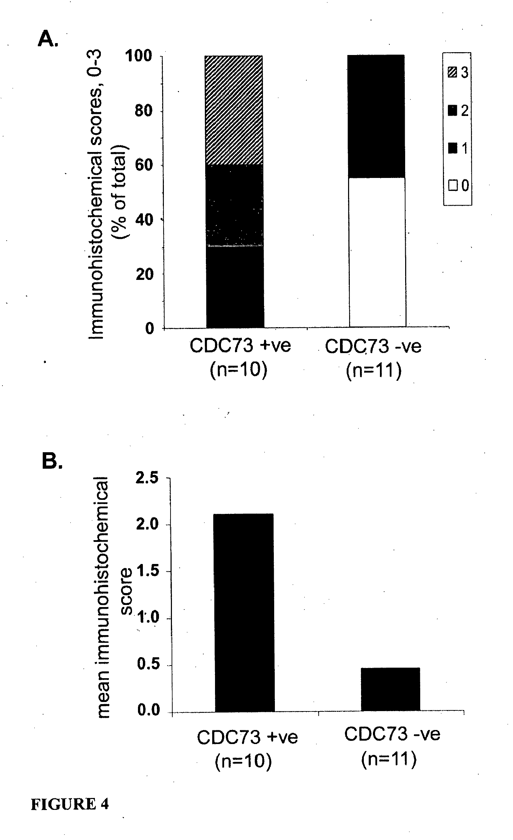

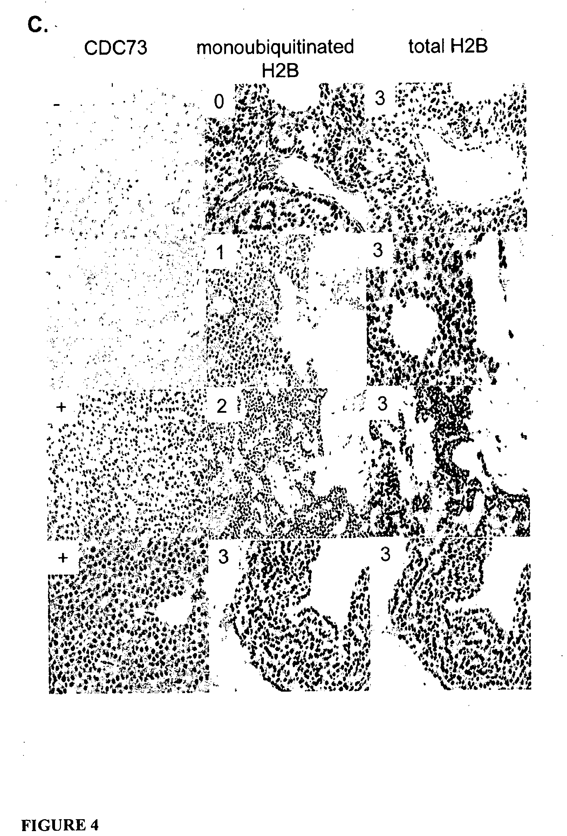

**Figure 4** demonstrates that H2B monoubiquitination is significantly reduced in parafibromin (CDC73) negative parathyroid tumours. Immunohistochemical staining and scoring for monoubiquitinated H2B and total H2B of 10 parafibromin positive parathyroid adenomas and 11 parafibromin negative carcinomas was carried out. The percentage of parafibromin positive or negative tumours exhibiting each immunohistochemical score was determined (A). The mean immunohistochemical score was also determined for parafibromin positive and negative tumours (B). A representative immunostained section of each score from two parafibromin negative and two parafibromin positive tumours is shown (C).

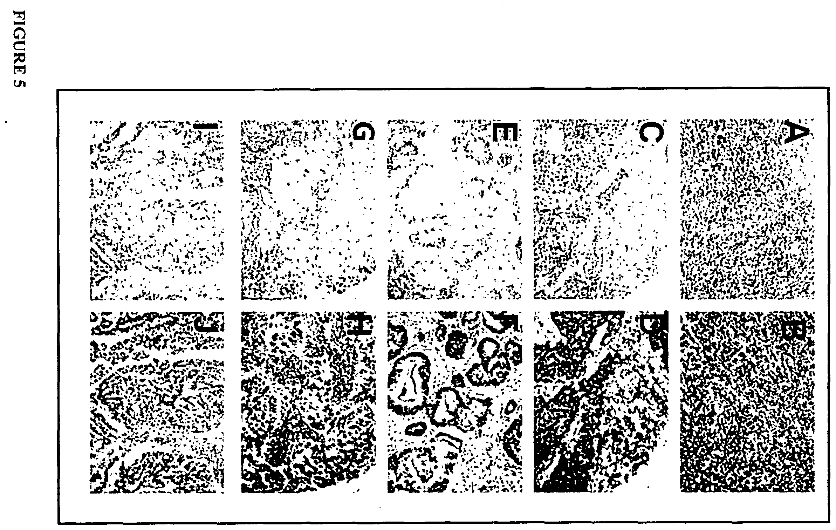

**Figure 5** shows immunohistochemical staining of specimens arrayed on commercial tissue arrays for monoubiquitinated histone 2B (*A, C, E, G, I*) and total histone 2B (*B, D, F, H, J*). Selected specimens are shown. Tumour types included melanoma (*A, B*), breast cancer (*C*, *D*), colorectal cancer (*E*, *F*), lung cancer (*G, H*) and ovarian cancer (*J*, *K*).

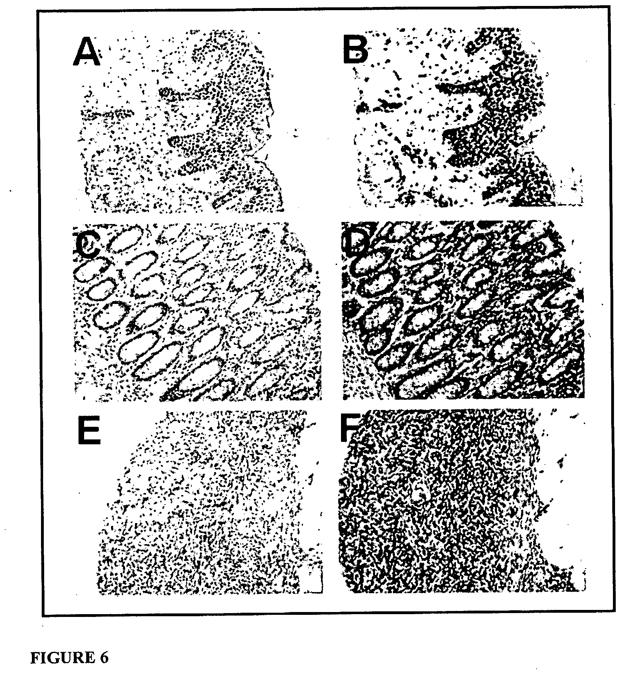

**Figure 6** shows immunohistochemical staining of normal tissue specimens arrayed on commercial tissue arrays for monoubiquitinated histone 2B (A, C, E) and total histone 2B (*B, D, F*). Selected specimens are shown. Tissue types included normal skin (*A, B*), normal colonic mucosa (*C, D*), and normal ovarian stromal tissue (*E, F*).

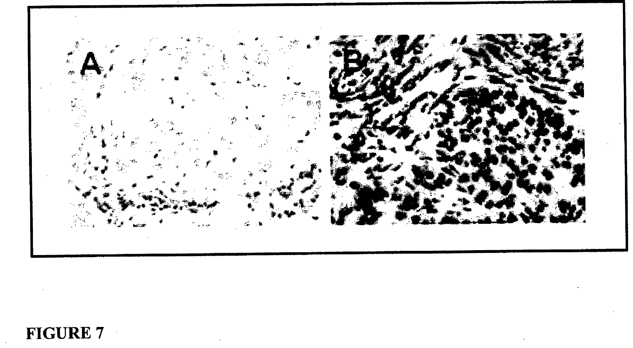

**Figure 7** shows immunohistochemical staining for (A) monoubiquitinated histone 2B and (B) total histone 2B in a BRCA1 mutant breast tumour.

### DEFINITIONS

As used in this application, the singular form "a", "an" and "the" include plural references unless the context clearly dictates otherwise. For example, the term "a histone protein" also includes a plurality of histone proteins.

As used herein, the term "comprising" means "including." Variations of the word "comprising", such as "comprise" and "comprises," have correspondingly varied meanings. Thus, for example, a method "comprising" a given step may consist exclusively of that step, or, may include one or more additional steps.

As used herein, the term "ubiquitination" encompasses both monoubiquitination and polyubiquitination.

As used herein, the terms "histone H2B", "histone 2B" and "H2B" are used interchangeably and have the same meaning unless otherwise indicated.

As used herein, an "agent" includes within its scope any natural or manufactured element or compound. Accordingly, the term includes, but is not limited to, any chemical elements and chemical compounds, nucleic acids, amino acids, polypeptides, proteins, antibodies and fragments of antibodies, and other substances that may be appropriate in the context of the invention.

As used herein, the term "administering" and variations of that term including "administer" and "administration", includes contacting, applying, delivering or providing a compound (e.g. a nucleic acid, polypeptide or antibody) to a cell by any appropriate means.

As used herein, the terms "antibody" and "antibodies" include IgG (including IgG1, IgG2, IgG3, and IgG4), IgA (including IgA1 and IgA2), IgD, IgE, or IgM, and IgY, whole antibodies, including single-chain whole antibodies, and antigen-binding fragments thereof. Antigen-binding antibody fragments include, but are not limited to, Fab, Fab' and F(ab')2, Fd, single-chain Fvs (scFv), single-chain antibodies, disulfide-linked Fvs (sdFv) and fragments comprising either a VL or VH domain. The antibodies may be from any animal origin. Antigen-binding antibody fragments, including single-chain antibodies, may comprise the variable region(s) alone or in combination with the entire or partial of the following: hinge region, CH1, CH2, and CH3 domains. Also included are any combinations of variable region(s) and hinge region, CH1, CH2, and CH3 domains. Antibodies may be monoclonal, polyclonal, chimeric, multispecific, humanized, and human monoclonal and polyclonal antibodies, which specifically bind the biological molecule.

As used herein, the term "specific for" refers to binding specificity. Accordingly, a molecule "specific for" another different molecule is one with binding specificity for that different molecule. For example, if molecule A is "specific for" molecule B, molecule A has the capacity to discriminate between molecule B and any other number of potential alternative binding partners. Accordingly, when exposed to a plurality of different but equally accessible molecules as potential binding partners, molecule A will selectively bind to molecule B and other alternative potential binding partners will remain substantially unbound by the reagent. In general, molecule A will preferentially bind to molecule B at least 10-fold, preferably 50-fold, more preferably 100-fold, and most preferably greater than 100-fold more frequently than other potential binding partners. Molecule A may be capable of binding to molecules that are not molecule B at a weak, yet detectable level. This is commonly known as background binding and is readily discernible from molecule B-specific binding, for example, by use of an appropriate control.

The term "*BRCA1* gene" as used herein is a reference to the human gene located at human 17q21 (e.g. base pair 41,196,311 to base pair 41,277,499). Non-limiting examples of human *BRCA1* sequences include those disclosed under NCBI Reference Sequence accession number NG_017013.1, and GenBank accession numbers U68041, AF005068, BC072418, BC085615 and BC046142.

The term "*BRCA2* gene" as used herein is a reference to the human gene located located at 13q12.3 (e.g. base pairs 32,889,616 to 32,973,808). Non-limiting examples of human *BRCA2* sequences include those dislosed under NCBI Reference Sequence accession number NM_000059, and GenBank accession numbers U43746, AY083934.

As used herein, a cancer "characterised" by a mutation or a combination of mutations in the *BRCA1* gene, the *BRCA2* gene, or both genes is intended to encompass any form of cancer whose occurrence is attributed at least in part, or, entirely to, mutations or other abnormalities in the *BRCA1* gene and/or the *BRCA2* gene.

Any description of prior art documents herein, or statements herein derived from or based on those documents, is not an admission that the documents or derived statements are part of the common general knowledge of the relevant art.

### DETAILED DESCRIPTION

The present inventors have identified that reduced histone protein ubiquitination can be indicative of cancer. Experimental data provided in the Examples of the present specification is indicative that the E3 ubiquitin ligase ring finger protein RNF20 is a binding partner of CDC73 (also referred to herein as parafibromin). Without limitation to a particular mechanism, CDC73 via its interaction with RNF20 is proposed to regulate monoubiquitination of histone proteins, a modification that is associated with actively transcribed chromatin. It is postulated that the dysregulation of histone protein ubiquitination may be a factor potentially causative of cancer.

Despite the association of histone protein monoubiquitination with actively transcribed chromatin (and hence increased transcription), the present inventors have unexpectedly identified that reduced levels of histone protein monoubiquitination in cells can serve as a diagnostic marker for cancer.

Hence, certain aspects of the present invention relate to methods for the diagnosis of cancer. The methods comprise measuring histone protein monoubiquitination in suspected cancer cells from a subject, wherein decreased levels of histone protein monoubiquitination in those cells compared to levels of histone protein monoubiquitination in non-cancerous cells is diagnostic of cancer. Also disclosed are kits for the diagnosis of cancer in a subject. The kits comprise components capable of measuring the level of histone protein monoubiquitination in cells from a subject. Also disclosed is the identification of agents that modify interactions between CDC73 and RNF20. By virtue of modifying these interactions, the agents may be capable of altering histone protein monoubiquitination mediated by CDC73/RNF20. This in turn may influence the transcription of certain genes involved in the emergence of cancer.

### Biomarkers

In the context of the present invention, a "biomarker" encompasses a biological molecule or a combination of biological molecules diagnostic and/or prognostic of cancer. Accordingly, the present invention provides biomarkers for the diagnosis and/or prognosis of cancer, also referred to hereinafter as "biomarker(s) of the invention".

Biomarkers of the invention comprise or consist of one or more ubiquitinated amino acid residue(s) of a histone protein. For the sake of clarity, a ubiquitinated amino acid residue is a residue which has one or more attached ubiquitin molecule(s). A ubiquitinated amino acid residue in accordance with the invention may therefore have a single attached ubiquitin residue or multiple attached ubiquitin residues Accordingly, a biomarker of the invention may comprise one or more monoubiquitinated amino acid residue(s) of a histone protein and/or one or more polyubiquitinated amino acid residue(s) of a histone protein. The ubiquitinated residue is preferably a lysine residue, although a biomarker of the invention may comprise other ubiquitinated amino acid residues (e.g. ubiquitinated threonine and/or serine).

Preferably, biomarkers of the invention comprise or consist of one or more monoubiquitinated amino acid residue(s) of a histone protein (i.e. one or more residues modified by the attachment of a single ubiquitin residue).

The histone protein may be any histone protein including, for example, histone proteins of the families H1/H5, H2A, H2B, H3, and H4. The histone protein may be a core histone protein or a linker histone protein.

In certain embodiments, the histone protein is a histone H2B (H2B) family protein. Non-limiting examples of H2B family proteins include histone H2B1 proteins (e.g. histone H2B type 1-A, histone H2B type 1-B, histone H2B type 1-C, histone H2B type 1-D, histone H2B type 1-E, histone H2B type 1-F, histone H2B type 1-G, histone H2B type 1-H, histone H2B type 1-I, histone H2B type 1-J, histone H2B type 1-K, histone H2B type 1-L, histone H2B type 1-M, histone H2B type 1-N, and histone H2B type 1-O); histone H2BF proteins (e.g. Histone H2B type F-S, Histone H2B type F-M, Histone H2B type W-T and Histone H2B type F-O), and histone H2B2 proteins (e.g. Histone H2B type 2-E).

The histone protein may be a mammalian histone protein. For example, the histone protein may be a bovine, equine, ovine, canine, feline, primate, or rodent histone protein. In other embodiments the histone protein is an avian histone protein.

In certain embodiments, the histone protein is a human histone protein. The human histone protein may be a human histone H2B protein. For example, the human histone H2B protein may have the amino acid sequence set forth in any one of GenBank accession numbers AAN59961, AAN06684-AAN06698, CAA11276-CAA11277, CAB06033, CAB06035, CAB02542-CAB02545, CAA40406, or CAA41051.

In certain embodiments, a biomarker of the invention comprises at least one ubiquitinated amino acid residue of a human histone H2B protein. The human histone H2B protein may comprise the amino acid sequence set forth in any of the GenBank accession numbers referred to in the preceding paragraph. In preferred embodiments, a biomarker of the invention comprises at least one monoubiquitinated amino acid residue of a human histone H2B protein. The monoubiquitinated residue may be a lysine residue at any position of the human histone H2B protein. For example, the monoubiquitinated residue may be a lysine residue at any one or more of positions 6, 12, 13, 16, 17, 21, 24, 25, 28, 29, 31, 35, 44, 47, 58, 109, 117, 121 or 126 of a human histone H2B protein of 126 amino acid residues. In particularly preferred embodiments, a biomarker of the invention comprises a monoubiquitinated lysine residue at position 121 of a human histone H2B protein. The skilled addressee will readily understand that in the case where a shortened human histone H2B polypeptide sequence is contemplated (i.e. a human histone H2B polypeptide sequence that is less than 126 amino acids long) the positions of the lysine residues referred to above (i.e. those of a 126 amino acid human histone H2B protein) will differ despite representing the same lysine residues. In such cases, biomarkers of the invention encompass any lysine residues corresponding to those at positions 6, 12, 13, 16, 17, 21, 24, 25, 28, 29, 31, 35, 44, 47, 58, 109, 117, 121 or 126 in a human histone H2B protein of 126 amino acid residues.

For example, in some cases human histone H2B protein sequences are provided without an initial methionine residue and are thus 125 amino acids in length (see, for example, Figure 3 on page r318 of Wells and McBride, (1989), "A comprehensive alignment of histones and histone genes", Nucleic Acids Res. 1989; 17 Suppl: r311-46). Accordingly, in a shortened human histone H2B protein provided without, for example, an initial methionine residue, biomarkers of the invention may comprise a monoubiquitinated lysine residue at any one or more of positions 5, 11, 12, 15, 16, 20, 23, 24, 27, 28, 30, 34, 43, 46, 57, 108, 116, 120 or 125. Preferably, the monoubiquitinated lysine residue is at position 120 of the shortened human histone H2B protein.

Although a biomarker of the invention may comprise one or more ubiquitinated amino acids residues from a single histone protein, it is also contemplated that biomarkers of the invention may comprise combinations of ubiquitinated residue(s) from different histone proteins. For example, the biomarker may comprise monoubiquitinated residue(s) of histone proteins from different families (e.g. histone protein H2A and histone protein H2B family member proteins), and/or monoubiquitinated residue(s) of different histone proteins from the same family (e.g. different histone protein 2B family member proteins).

### Detection of biomarkers

A biomarker of the invention may be detected using any suitable method known in the art. Given that histone proteins are predominantly internal proteins and generally not expressed on the surface of cells, detection methods suitable for identifying internal cellular proteins are preferred.

A biomarker of the invention may be detected using suitable antibodies. The antibodies will have binding specificity for given ubiquitinated histone protein(s). Preferably, the histone protein(s) is/are monoubiquitinated. It will be understood that antibodies capable of "binding specifically" to a target ubiquitinated histone protein are "specific for" that protein, and are capable of binding to the target protein with a significantly higher affinity in comparison to other unrelated proteins (e.g. a non-ubiquitinated histone protein). Accordingly, an antibody that binds specifically to a target ubiquitinated histone protein is an antibody with the capacity to discriminate between that target protein and any other number of potential alternative binding partners. Accordingly, when exposed to a plurality of different but equally accessible proteins as potential binding partners, an antibody specific for a target ubiquitinated histone protein will selectively bind to that target protein and other alternative potential binding partners will remain substantially unbound by the antibody. In general, an antibody specific for a target ubiquitinated histone protein will preferentially bind to the target protein at least 10-fold, preferably 50-fold, more preferably 100-fold, and most preferably greater than 100-fold more frequently than other potential binding partners that are not target molecules. An antibody specific for a target ubiquitinated histone protein may be capable of binding to other non-target molecules at a weak, yet detectable level. This is commonly known as background binding and is readily discernible from specific binding, for example, by use of an appropriate control.

Reaction conditions (e.g. concentration of antibody, incubation time, pH, temperature etc) to facilitate binding of antibodies to a given ubiquitinated histone protein will depend primarily on the antibody utilised and the specific protein, and may be readily determined using methods known in the art (see, for example, Ausubel et al. (eds), (1994), "Current Protocols in Molecular Biology", Vol. 1, John Wiley & Sons, Inc., New York; Coligan et al., (eds), (2008), "Current protocols in Immunology", John Wiley and Sons, Inc.; and Bonifacino et al., (eds) (2007), "Current protocols in Cell Biology", John Wiley and Sons, Inc., New York).

Antibodies capable of binding specifically to a target ubiquitinated histone protein can be generated using methods known in the art.

For example, a monoclonal antibody specific for a target protein of interest, typically containing Fab portions, may be prepared using the hybridoma technology described in Harlow and Lane (eds), (1988), "Antibodies - A Laboratory Manual", Cold Spring Harbor Laboratory, NY.

In essence, in the preparation of monoclonal antibodies directed toward a target protein, any technique that provides for the production of antibodies by continuous cell lines in culture may be used. These include the hybridoma technique originally developed by Kohler et al., (1975), "Continuous cultures of fused cells secreting antibody of predefined specificity", Nature, 256:495-497, as well as the trioma technique, the human B-cell hybridoma technique (see Kozbor et al., (1983), "The Production of Monoclonal Antibodies From Human Lymphocytes", Immunology Today, 4:72-79), and the EBV-hybridoma technique to produce human monoclonal antibodies (see Cole et al., (1985), in "Monoclonal Antibodies and Cancer Therapy", 77-96, Alan R. Liss, Inc.). Immortal, antibody-producing cell lines can be created by techniques other than fusion, such as direct transformation of B lymphocytes with oncogenic DNA, or transfection with Epstein-Barr virus (see, for example, Schreier et al., (1980), "Hybridoma Techniques", Cold Spring Harbor Laboratory; Hammerling et al., (1981), "Monoclonal Antibodies and T-cell Hybridomas", Elsevier/North-Holland Biochemical Press, Amsterdam; and Kennett et al., (1980), "Monoclonal Antibodies", Plenum Press).

In summary, a means of producing a hybridoma from which the monoclonal antibody is produced, a myeloma or other self-perpetuating cell line is fused with lymphocytes obtained from the spleen of a mammal hyperimmunised with a recognition factor-binding portion thereof, or recognition factor, or an origin-specific DNA-binding portion thereof. Hybridomas producing a monoclonal antibody specific for the target protein are identified by their ability to immunoreact with the antigens present in that protein. For example, hybridomas producing a monoclonal antibody specific for the target protein may be identified by an ability to immunoreact with a branched peptide corresponding to the conjugation site of ubiquitin on given amino acid residue(s) of a histone protein.

A monoclonal antibody specific for a target protein can be produced by initiating a monoclonal hybridoma culture comprising a nutrient medium containing a hybridoma that secretes antibodies of the appropriate antigen specificity. The culture is maintained under conditions and for a time period sufficient for the hybridoma to secrete the antibody molecules into the medium. The antibody-containing medium is then collected. The antibody molecules can then be further isolated using known techniques.

Similarly, there are various procedures known in the art which may be used for the production of polyclonal antibodies. For the production of polyclonal antibodies specific for a target protein, various host animals can be immunized by injection with the protein including, but not limited to, rabbits, chickens, mice, rats, sheep, goats, and the like. Further, the protein can be conjugated to an immunogenic carrier (e.g. bovine serum albumin (BSA) or keyhole limpet hemocyanin (KLH)). Also, various adjuvants may be used to increase the immunological response including, but not limited to, Freund's (complete and incomplete), mineral gels such as aluminium hydroxide, surface active substances such as rysolecithin, pluronic polyols, polyanions, peptides, oil emulsions, keyhole limpet hemocyanins, dinitrophenol, and potentially useful human adjuvants such as BCG (bacille Calmette-Guerin) and Corynebacterium parvum.

Screening for the desired antibody can be accomplished by a variety of techniques known in the art. Suitable assays for immunospecific binding of antibodies include, but are not limited to, radioimmunoassays, ELISAs (enzyme-linked immunosorbent assay), sandwich immunoassays, immunoradiometric assays, gel diffusion precipitation reactions, immunodiffusion assays, in situ immunoassays, Western blots, precipitation reactions, agglutination assays, complement fixation assays, immunofluorescence assays, protein A assays, immunoelectrophoresis assays, and the like (for description of such techniques see, for example, Ausubel et al., (eds), (2000-2010), "Current Protocols in Molecular Biology", Vol. 1, John Wiley & Sons, Inc., New York). Antibody binding may be detected by virtue of a detectable label on the primary antibody. Alternatively, the antibody may be detected by virtue of its binding with a secondary antibody or reagent which is appropriately labelled. A variety of methods for detecting binding events in an immunoassay are known in the art, and are included in the scope of the invention.

In terms of obtaining a suitable amount of an antibody, one may manufacture the antibodies using batch fermentation with serum free medium. After fermentation the antibody may be purified via a multistep procedure incorporating chromatography and viral inactivation/removal steps. For instance, the antibody may be first separated by Protein A affinity chromatography and then treated with solvent/detergent to inactivate any lipid enveloped viruses. Further purification, typically by anion and cation exchange chromatography, may be used to remove residual proteins, solvents/detergents and nucleic acids. The purified antibody may be further purified and formulated into 0.9% saline using gel filtration columns. The formulated bulk preparation may then be sterilised and viral filtered and dispensed.

In certain embodiments, an antibody specific for a ubiquitinated histone H2B protein may be used to detect a biomarker of the invention. Preferably, the protein is a human histone H2B protein. The human histone H2B protein may, for example, have the amino acid sequence set forth in any one of GenBank accession numbers AAN59961, AAN06684-AAN06698, CAA11276-CAA11277, CAB06033, CAB06035, CAB02542-CAB02545, CAA4046, or CAA41051. The antibody may bind specifically to at least one monoubiquitinated amino acid residue of the human histone H2B protein. The monoubiquitinated residue may be a lysine residue. For example, the monoubiquitinated residue may be a lysine residue at any one or more of residues 6, 12, 13, 16, 17, 21, 24, 25, 28, 29, 31, 35, 44, 47, 58, 109, 117, 121 or 126 of a human histone H2B protein of 126 amino acids (or a corresponding lysine residue in a human histone H2B protein of shorter length). In particularly preferred embodiments, the antibody binds specifically to a monoubiquitinated lysine residue at position 121 of a human histone H2B protein of 126 amino acids (or a corresponding lysine residue in a shortened human histone H2B protein).

Antibodies suitable for this purpose can be generated using methods known in the art (see paragraphs above) or obtained from commercial sources. A non-limiting example of a suitable commercially available antibody is a mouse monoclonal anti-ubiquitinated histone H2B (NRO3) sold by MediMabs (Montreal, Canada) under catalogue number MM-0029.

Biomarkers of the invention may be detected in cells of a given subject using any suitable assay including, but not limited to, those described in the Examples of the present specification.

For example, biomarkers of the invention may be detected using standard protein immunblotting assays. Immunoblotting assays are known in the art and described, for example, in the Examples of the present specification, and in Gallagher (2001), "Immunoblot Detection" (Unit 10.10) in "Current Protocols in Protein Science", Coligan et al. (eds)., (1999-2010), John Wiley & Sons, Inc., New York. In general, immunoblotting relies on the use of ligands (e.g. antibodies) to identify target proteins (i.e. ubiquitinated histone proteins) among a number of unrelated protein species. The protein target is identified by antigen-antibody (or protein-ligand) specific reactions. Proteins from a given sample of cells are typically separated by electrophoresis and transferred onto a membrane (e.g. a nitrocellulose membrane). The membrane may be treated with a primary antibody that binds specifically to the target protein followed by a secondary labeled antibody that binds specifically to the primary antibody. The secondary antibody may be labeled, for example, with radioisotopes or with enzymes. If the membrane is treated with a ligand that is not an antibody, the reaction may be visualised using a ligand that is directly labeled.

In certain embodiments, a dot blot may be used to detect biomarkers of the invention in which proteins from a given sample of cells are not separated by electrophoresis but are spotted directly onto the membrane for detection.

Additionally or alternatively, biomarkers of the invention may be detected using immunohistochemical techniques. These techniques are known in the art and are described, for example, in the Examples of the present specification and in Watkins (2009), "Immunohistochemistry" (Unit 12.16) in "Current Protocols in Cytometry", Robinson et al., (eds.), (1999-2010), John Wiley & Sons, Inc., New York. In general, immunohistochemistry relies on the detection of target proteins (e.g. monoubiquitinated histone proteins) in tissue sections using antibodies that bind specifically to the target protein. For example, a primary antibody may be applied to an appropriately prepared tissue section (e.g. formalin-fixed, sectioned and mounted). Primary antibodies bound to target protein can be visualized by way of detectable labels (e.g. fluorescent dye, enzyme, or colloidal gold.) which are preferably conjugated to a secondary antibody specific for the primary antibody. Upon application of the secondary antibody, primary antibody bound to target proteins in cells of the section can be visualised using appropriate means (e.g. fluorescent microscopy and the like).

Additionally or alternatively, biomarkers of the invention may be detected using fluorescence activated cell sorting (FACS), otherwise known as flow cytometry. Flow cytometry techniques are known in the art. The general principles of flow cytometry, and assays for the preparation of cells for use in flow cytometry are described, for example, in Robinson et al., (eds.), (1999-2010), "Current Protocols in Cytometry", John Wiley & Sons, Inc., New York; and Coligan et al., (eds.), (1999-2010) "Current Protocols in Immunology", (see for example, Chapter 5). In general, a population of cells comprising ubiquitinated histone proteins may be subjected to permeabilisation allowing primary antibodies specific for a given target protein to enter the cell interior whereupon one or more internal target proteins may be bound by the antibodies. After removal of unbound primary antibodies from the cells, binding events between primary antibodies and target proteins may be identified by virtue of a fluorescent label conjugated to the primary antibodies. Alternatively, binding events between primary antibodies and target proteins may be detected by the application of a fluorescently labelled secondary antibody specific for the first antibody. In either case, the fluorescent label is detected by passing the cells through a flow cytometer.

The skilled addressee will understand that the specific methods for detecting biomarkers of the invention provided above are exemplary only and do not limit the scope of the invention described herein.

### Diagnostic and prognostic methods

In certain aspects, the invention provides methods for the diagnosis and/or prognosis of cancer in a subject. The methods comprise measuring histone protein ubiquitination in cells of the subject.

The methods may be performed *in vivo*, *in vitro,* or *ex vivo.* The subject may be an individual of any mammalian species including, but not limited to, members of the genus ovine, bovine, equine, porcine, feline, and canine, primates (e.g. humans), and rodents. Preferably, the subject is a human. Alternatively, the subject may be an avian species.

The subject may be a healthy individual, an individual suspected to be suffering from cancer, or an individual known to be suffering from cancer.

Cells of the subject for use in diagnostic and/or prognostic methods of the invention may be provided in the form of a biological sample. It will be understood that a "biological sample" as contemplated herein includes a sample that is modified from its original state, for example, by purification, dilution, embedding in a wax block, or the addition of any other component or components.

Non-limiting examples of biological samples include whole blood or a component thereof (e.g. plasma, serum), urine, saliva, lymph, bile fluid, sputum, tears, cerebrospinal fluid, bronchioalveolar lavage fluid, synovial fluid, semen, ascitic tumour fluid, breast milk, tissue, and pus. In one embodiment, the biological sample is a biopsy obtained from a tumour or a suspected tumour.

The cells may have been obtained from a subject previous to performing the methods of the invention. For example, the cells may be cultured cells, stored cells (e.g. frozen), or fresh cells.

In preferred embodiments, the cells of the subject are suspected of being cancerous cells. For example, the cells may be derived from tissue in the subject that is suspected of being cancerous (e.g. a suspected tumour, or an organ or gland suspected of comprising cancerous cells). In certain embodiments, the cells are derived from a parathyroid gland suspected of comprising cancerous cells.

Diagnostic and/or prognostic methods of the invention comprise measuring histone protein ubiquitination in the cells of a subject.

For example, one aspect of the invention provides a method for diagnosing cancer in a subject, the method comprising measuring histone protein monoubiquitination in a first cell of the subject, wherein decreased histone protein monoubiquitination in said first cell compared to that of a second non-cancerous cell is diagnostic of cancer.

It will be understood that measuring histone protein monoubiquitination in a "first cell" of the subject encompasses the act of measuring histone protein monoubiquitination in multiple cells (e.g. a first cell population). The level of histone protein monoubiquitination in a "first cell" of the subject may therefore be determined, for example, by deriving an average or mean level of histone protein monoubiquitination in multiple "first cells". Likewise, measuring histone protein monoubiquitination in a "second non-cancerous cell" encompasses the act of measuring histone protein monoubiquitination in multiple non-cancerous cells (e.g. a second cell population of non-cancerous cells). The level of histone protein monoubiquitination in a "second non-cancerous cell" of the subject may therefore be determined, for example, by deriving an average or mean level of histone protein monoubiquitination in multiple "second non-cancerous cells".

In certain embodiments of the invention, the level of histone protein monoubiquitination may be measured in a first cell population from a biological sample of potentially cancerous cells (e.g. a biopsy specimen taken from a suspected tumour in a given tissue of the subject) and compared to the level of histone protein monoubiquitination in a second cell population from a biological sample of non-cancerous cells (e.g. a biopsy specimen that is suspected or known to be made up of non-cancerous cells taken from the same tissue of the subject, and/or a different tissue of the subject). If a decrease in the level of histone protein monoubiquitination in the first cell population is detected compared to the second cell population, a positive diagnosis for cancer is provided. Alternatively, if the level of histone protein monoubiquitination in the first cell population is not decreased (or is increased) compared to the second cell population, a negative diagnosis for cancer is provided.

The level of histone protein ubiquitination in a given cell may be assessed by detection of one or more biomarkers of the invention as described in the sections above entitled "Biomarkers" and "Detection of Biomarkers".

In general, the detection of a decrease in histone protein monoubiquitination of cells from the subject may be indicative that the cell being tested is a cancerous cell. It will be understood that a "decrease" in histone protein monoubiquitination may be characterised by a reduction rather than a complete loss of histone protein monoubiquitination. However, a complete loss of histone protein monoubiquitination is not excluded.

A "decrease" in histone protein monoubiquitination in cancerous cells of the subject may be determined, for example, by comparison to levels of histone protein monoubiquitination in those cells to levels of histone protein monoubiquitination in a reference population of non-cancerous (normal or benign disease) cells. Accordingly, a reduction in the level of histone protein monoubiquitination in the cells tested compared to levels in the reference population of cells indicates that the tested cells are cancerous (i.e. a positive diagnosis for cancer is provided). Conversely, if the level of histone protein monoubiquitination in the cells tested is not decreased relative to the reference population of cells (or increased over levels in the reference population of cells), a negative diagnosis for cancer is provided.

In certain embodiments, the reference population of cells are non-cancerous cells derived from the subject under diagnosis. In other embodiments, the reference population of cells are non-cancerous cells derived from a different individual to the subject under diagnosis. Preferably, the different individual belongs to the same species as the subject under diagnosis, and more preferably the different individual is genetically related to the subject under diagnosis. In other embodiments, the reference population of cells are non-cancerous cells derived from a combination of different individuals belonging to the same species as the subject under diagnosis, such that a standard range of histone protein monoubiquitination in non-cancerous cells of individuals belonging to the same species is used as a basis for the comparison.

Total H2B expression may be constant or substantially constant in non-cancerous and cancerous cells. Hence, it may be used as a means of standardising measurements of monoubiquitination levels in various different cell populations including cancerous and non-cancerous cells types. Accordingly, in certain embodiments a decrease in histone protein monoubiquitination in cells of the subject may be detected by comparison with the level of total histone H2B expression (i.e. normalisation on the basis of total H2B expression).

The degree of histone protein ubiquitination in a given cell can be determined using methods known in the art including, but not limited to, those described in the section above entitled "Detecting Biomarkers" and/or those described in the Examples of the present specification.

In accordance with diagnostic and/or prognostic methods of the invention, a positive diagnosis for cancer may be indicated by a decrease in histone protein monoubiquitination in a given cell or cell population from a subject. As noted above, a decrease in histone protein monoubiquitination may be determined, for example, by comparison to levels of histone protein monoubiquitination in non-cancerous cells. In certain embodiments of the invention, a positive diagnosis for cancer may be indicated by a decrease of at least 5%, 10%, 15%, 20% or 25% in histone protein monoubiquitination in the cells being tested compared to the reference cells. In other embodiments of the invention, a positive diagnosis for cancer may be indicated by a decrease of at least 30%, 35%, 40%, 45% or 50% in histone protein monoubiquitination in the cells being tested compared to the reference cells. In further embodiments of the invention, a positive diagnosis for cancer may be indicated by more than a 50% decrease in histone protein monoubiquitination in the cells being tested compared to the reference cells.

In preferred embodiments, diagnostic and/or prognostic methods of the invention are used to detect histone protein ubiquitination in a given cell or cell population from a subject. The histone protein may be a core histone protein or a linker histone protein. The histone protein may be any histone protein including, for example, histone proteins of the families H1/H5, H2A, H2B, H3, and H4.

The histone protein may be a histone H2B (H2B) family protein. Non-limiting examples of H2B family proteins include histone H2B1 proteins (e.g. histone H2B type 1-A, histone H2B type 1-B, histone H2B type 1-C, histone H2B type 1-D, histone H2B type 1-E, histone H2B type 1-F, histone H2B type 1-G , histone H2B type 1-H, histone H2B type 1-I, histone H2B type 1-J, histone H2B type 1-K, histone H2B type 1-L, histone H2B type 1-M, histone H2B type 1-N, and histone H2B type 1-O); histone H2BF proteins (e.g. Histone H2B type F-S, Histone H2B type F-M, Histone H2B type W-T and Histone H2B type F-O), and histone H2B2 proteins (e.g. Histone H2B type 2-E).

The histone protein may be a mammalian histone protein. For example, the histone protein may be a bovine, equine, ovine, canine, feline, primate, or rodent histone protein. In other embodiments the histone protein is an avian histone protein.
In certain embodiments, the histone protein is a human histone protein. The human histone protein may be a human histone H2B protein. For example, the human histone H2B protein may have the amino acid sequence set forth in any one of GenBank accession numbers AAN59961, AAN06684-AAN06698, CAA11276-CAA11277, CAB06033, CAB06035, CAB02542-CAB02545, CAA4046, or CAA41051.

In preferred embodiments, diagnostic and/or prognostic methods of the invention are used to detect at least one ubiquitinated amino acid residue of a histone H2B protein. The histone H2B protein may be a human histone H2B protein. The monoubiquitinated residue may be a lysine residue. For example, the monoubiquitinated residue may be a lysine residue at any one or more of positions 6, 12, 13, 16, 17, 21, 24, 25, 28, 29, 31, 35, 44, 47, 58, 109, 117, 121 or 126 of a human histone H2B protein of 126 amino acids (or a corresponding lysine residue in a human histone H2B protein of shorter length). In particularly preferred embodiments, the antibody binds specifically to a monoubiquitinated lysine residue at position 121 of a human histone H2B protein of 126 amino acids (or a corresponding lysine residue in a shortened human histone H2B protein).

Diagnostic methods of the invention may be used to make a positive or negative diagnosis of cancer in a subject. Additionally or alternatively, the methods may be used for prognostic purposes. For example, the level of histone protein monoubiquitination (e.g. monoubiquitination of lysine at residue 121 of histone protein H2B) in cancerous cells of a subject may be predictive of a particular disease state and may thus be used for prognostic purposes. For example, decreased histone H2B monoubiquitination at lysine residue 121 may be linked with tumours that are more aggressive, tumours that have a worse prognosis in the context of patient survival, tumours that are larger, and/or tumours that are more likely to invade and metastasise.

In certain embodiments, diagnosis and/or prognosis in accordance with the methods of the invention may be specific to a cancer type including, but not limited to, any one or more of parathyroid cancer, and/or cancer of the colon, breast, lung, prostate, ovary, brain, and/or skin. Additional non-limiting examples of cancer type/s that may be diagnosed and/or prognosed in accordance with the methods of the invention include cancer of the pancreas, liver, oesophagus, thyroid gland, endometrium, pituitary gland, adrenal gland, breast, and/or prostate gland. Furthermore, the diagnostic and/or prognostic methods of the invention may identify tumours that appear benign but have the potential to become malignant.

In some embodiments, the cancer may be parathyroid cancer, breast cancer, colorectal cancer, lung cancer, melanoma, adrenal cancer, and/or ovarian cancer.

The cancer may be characterised by a mutation or a combination of mutations in the *BRCA1* gene (breast cancer 1, early onset), the *BRCA2* gene (breast cancer 2, early onset), or both the *BRCA1* and *BRCA2* genes.

The term "*BRCA1* gene" as used herein is a reference to the human gene located at human 17q21 (e.g. base pair 41,196,311 to base pair 41,277,499). Non-limiting examples of human *BRCA1* sequences include those disclosed under NCBI Reference Sequence accession number NG_017013.1, and GenBank accession numbers U68041, AF005068, BC072418, BC085615 and BC046142.

The term "*BRCA2* gene" as used herein is a reference to the human gene located located at 13q12.3 (e.g. base pairs 32,889,616 to 32,973,808). Non-limiting examples of human *BRCA2* sequences include those dislosed under NCBI Reference Sequence accession number NM_000059, and GenBank accession numbers U43746, AY083934.

A cancer "characterised" by a mutation or a combination of mutations in the *BRCA1* gene, the *BRCA2* gene, or both genes is intended to encompass any form of cancer whose occurrence is attributed at least in part, or, entirely to, mutations or other abnormalities in the *BRCA1* gene and/or the *BRCA2* gene.

The mutation or mutations may, for example, lead to the production of a truncated version of the BRCA1 protein or the BRCA2 protein, or reduce/prevent translation of a BRCA1 or BRCA2 protein from one gene copy. Other non-limiting examples of applicable mutations in the BRCA1 and BRCA2 genes/proteins include conservative substitutions, non-conservative substitutions, missense mutations, nonsense mutations, frameshift mutations, readthrough, mutations, promoter mutations, regulatory mutations, deletions, insertions, inversions, splice mutations and duplications.

Specific and non-limiting examples of applicable mutations in the *BRCA1* and *BRCA2* genes include, for example, those listed in the kConFab database (www.kconfab.org/Progress/Mutations.aspx), and the "UMD" Universal Mutation Database (see http://www.umd.beBRCA1; and http://www.umd.be/BRCA2).

Diagnostic and prognostic methods of the invention may be used in combination with any other techniques suitable for similar purposes. For example, the methods may be used in combination with standard assays for cancer diagnosis. In certain embodiments, the methods are used in combination with assays for detecting the level of calcium and/or parathyroid hormone in the blood.

### Kits

Here disclosed are kits for the diagnosis and/or prognosis of cancer. The kits comprise at least one reagent that specifically binds to a ubiquitinated histone protein. The Kits may be used to perform the diagnostic and prognostic methods of the invention.

The histone protein may be any histone protein including, for example, histone proteins of the families H1/H5, H2A, H2B, H3, and H4. Preferably, the histone protein is a histone H2B family protein. The histone protein may be a mammalian histone protein (e.g. a human histone protein). Accordingly, the histone protein may be a human histone H2B protein. The human histone H2B protein may have the amino acid sequence set forth in any one of GenBank accession numbers AAN59961, AAN06684-AAN06698, CAA11276-CAA11277, CAB06033, CAB06035, CAB02542-CAB02545, CAA4046, or CAA41051.

In preferred embodiments, the reagent binds specifically to a monoubiquitinated amino acid residue of a human histone H2B protein. The monoubiquitinated residue may be a lysine residue. For example, the monoubiquitinated residue may be a lysine residue at any one or more of positions 6, 12, 13, 16, 17, 21, 24, 25, 28, 29, 31, 35, 44, 47, 58, 109, 117, 121 or 126 of a human histone H2B protein of 126 amino acids (or a corresponding lysine residue in a human histone H2B protein of shorter length).In particularly preferred embodiments, the antibody binds specifically to a monoubiquitinated lysine residue at position 121 of a human histone H2B protein of 126 amino acids (or a corresponding lysine residue in a shortened human histone H2B protein). The Kits may be used to make a positive or negative diagnosis of cancer in a subject. Additionally or alternatively, the kits may be used for prognostic purposes. For example, the level of histone protein ubiquitination (e.g. monoubiquitination of lysine at residue 121 of histone protein H2B) in cancerous cells of a subject may be predictive of a particular disease state and may thus be used for prognostic purposes.

In certain embodiments, the kits may be used for the diagnosis and/or prognosis of a cancer type selected from any one or more of parathyroid cancer and/or cancer of the colon, breast, lung, prostate, ovary, brain, and/or skin. Additional non-limiting examples of cancer type/s that may be diagnosed and/or prognosed in accordance with the methods of the invention include cancer of the pancreas, liver, oesophagus, thyroid gland, endometrium, pituitary gland, adrenal gland, breast, and/or prostate gland. Furthermore, the diagnostic and/or prognostic methods of the invention may identify tumours that appear benign but have the potential to become malignant.

Kits here disclosed comprise at least one reagent that specifically binds to a ubiquitinated histone protein. In certain embodiments, the reagent is an antibody. In particularly preferred embodiments, the antibody binds specifically to a monoubiquitinated lysine residue at position 121 of a human histone H2B protein. Antibodies suitable for this purpose can be generated using methods known in the art (see section above entitled "Detection of Biomarkers") or obtained from commercial sources. A non-limiting example of a suitable commercially available antibody is mouse monoclonal anti-ubiquitinated histone H2B (NRO3) sold by MediMabs (Montreal, Canada) under catalogue number MM-0029.

The kits may include other components required to conduct the methods of the present invention, such as secondary antibodies, enzymatic substrates, buffers, and/or diluents. For example, the kit may include an HRP- or ALP-conjugated secondary antibody capable of binding to a primary antibody specific for a ubiquitinated histone protein, along with the appropriate enzyme substrate.

Kits here disclosed may be used to perform protein immunoblotting, immunohistochemical analyses and/or flow cytometry.

### Screening methods

The present inventors have identified that RNF20 is a binding partner of CDC73. CDC73 is proposed herein to regulate ubiquitination of histone proteins through its interaction with RNF20, a modification associated with actively transcribed chromatin.

Certain aspects here disclosed relate to methods of screening for agents that reduce or augment binding between CDC73 and RNF20. The methods comprise administering a candidate agent to a population of cells comprising CDC73 and RNF20, analysing binding interactions between CDC73 and RNF20 in cells of the population, and, comparing the binding interactions with those of a second (control) cell population to which the candidate agent has not been administered.

It will be understood that a "binding interaction" between CDC73 and RNF20 in a given cell population encompasses both the number of binding interactions and the strength of binding interactions. Accordingly, binding interactions between CDC73 and RNF20 may comprise, for example, quantifying the number of binding interactions between CDC73 and RNF20 in cells of a given population and/or measuring the affinity of binding between CDC73 and RNF20 in cells of a given population.

Accordingly, in an exemplary embodiment, a first population of cells may be cultured in the presence of a given candidate agent under suitable conditions. If desirable, various duplicate cultures may be utilised to test the candidate agent at a range of different concentrations. A second identical or substantially identical population of cells may be cultured in the absence of the candidate agent under identical or substantially identical conditions. Cells from each population may be harvested after a suitable time period and prepared for protein analysis. Interactions between CDC73 and RNF20 in the presence and in the absence of a given candidate agent may be analysed by a variety of suitable methods including, for example, co-immunoprecipitation, affinity chromatography, mass spectroscopy, tandem affinity purification and protein microarrays.

Using the methods described above, an agent may be identified that is an agonist of CDC73 and RNF20 binding interactions. Agents which are agonists augment the number and/or strength of binding interactions between CDC73 and RNF20. Alternatively, an agent may be identified that is an antagonist of CDC73 and RNF20 binding interactions. Agents which are antagonists decrease the number and/or strength of binding interactions between CDC73 and RNF20.

It will be appreciated that the techniques described above are merely examples of the types of methods that may be utilised to identify agents that reduce or augment binding between CDC73 and RNF20. Other suitable methods will be known by persons skilled in the art and are within the scope of the invention.

As noted above, CDC73 is proposed herein to regulate ubiquitination of histone proteins through its interaction with RNF20, a modification associated with actively transcribed chromatin. Certain aspects here disclosed relate to methods of screening for agents that modulate ubiquitination of a histone protein. The methods comprise determining whether a candidate agent reduces or augments binding between CDC73 and RNF20 which may be determined, for example, using the methods described in the preceding paragraphs.

The histone protein may be any histone protein including, for example, histone proteins of the families H1/H5, H2A, H2B, H3, and H4. Preferably, the histone protein is a histone H2B family protein. The histone protein may be a mammalian histone protein (e.g. a human histone protein). Accordingly, the histone protein may be a human histone H2B protein. The human histone H2B protein may have the amino acid sequence set forth in any one of GenBank accession numbers AAN59961, AAN06684-AAN06698, CAA11276-CAA11277, CAB06033, CAB06035, CAB02542-CAB02545, CAA4046, or CAA41051.

In preferred embodiments, the agent modulates monoubiquitination of an amino acid residue of a human histone H2B protein. The residue may be a lysine residue. For example, the residue may be a lysine residue at any one or more of positions 6, 12, 13, 16, 17, 21, 24, 25, 28, 29, 31, 35, 44, 47, 58, 109, 117, 121 or 126 of a human histone H2B protein of 126 amino acids (or a corresponding lysine residue in a human histone H2B protein of shorter length).

An agent that "modulates" ubiquitination of a histone protein is one which has the effect of increasing or reducing ubiquitination of the histone protein compared to the level of ubiquitination that occurs in the absence of the agent under otherwise identical or substantially identical conditions. As noted in the preceding sections, the present inventors have identified that monoubiquitination of histone proteins is decreased in cancer cells. Hence, agents that modulate the ubiquitination of histone proteins by influencing binding interactions between CDC73 and RNF20 may be potentially relevant to the incidence and/or treatment of cancer.

Candidate agents for use in the screening methods of the invention may be derived from any source.

For example, the candidate agent may be naturally occurring or synthetic.

Potential candidate agents may be generated for use in the screening methods of the invention by a number of techniques known to those skilled in the art. For example, methods such as X-ray crystallography and nuclear magnetic resonance spectroscopy may be used to model the structure of CDC73 and/or RNF20, thus facilitating the design of potentially useful agents using computer-based modelling. Various forms of combinatorial chemistry may also be used to generate putative candidate agents.

A candidate agent may be of any molecular weight, for example, at least about 100, 200, 300, 400, 500, 750, 1000, 2000, 3000, 4000, 5000, 7000, 9000, 10000, 20000, 30000, 40000, 50000, 60000, 70000, 80000, 90000, or 100000 daltons.

A candidate agent can be any chemical compound, non-limiting examples of which include amino acids, nucleic acids, peptide nucleic acids, lipids, polypeptides, carbohydrates, and nucleosides other non-limiting examples include, peptidomimetics (e.g. peptoids), amino acid analogues, polynucleotides, polynucleotide analogues, nucleotides, nucleotide analogues, metabolites, metabolic analogues, and organic or inorganic compounds (including heteroorganic and organometallic compounds).

In certain embodiments high-throughput methods are used to screen large libraries of chemicals. Such libraries of candidate compounds can be generated or purchased from commercial sources. For example, a library can include 10,000, 50,000, or 100,000 or more unique compounds. By way of example only, a library may be constructed from heterocycles including benzimidazoles, benzothiazoles, benzoxazoles, furans, imidazoles, indoles, morpholines, naphthalenes, piperidines, pyrazoles, pyridines, pyrimidines, pyrrolidines, pyrroles, quinolines, thiazoles, thiphenes, and triazines. A library may comprise one or more classes of chemicals, for example, those described in Carrell et al., (1994), Angew. Chem. Int. Ed. Engl. 33:2059; Carell et al., (1994), Angew. Chem. Int. Ed. Engl. 33:2061; Cho et al., (1993), Science 261:1303-1305; DeWitt et al., (1993), Proc. Natl. Acad. Sci. U.S.A. 90:6909-6913; Erb et al., (1994), Proc. Natl. Acad. Sci. USA 91:11422-11426; Gallop et al., (1994), J. Med. Chem. 37:1233-1251; and Zuckermann et al., (1994), J. Med. Chem. 37:2678-2685.

It will be appreciated by persons skilled in the art that numerous variations and/or modifications may be made to the invention as shown in the specific embodiments. The present embodiments are, therefore, to be considered in all respects as illustrative and not restrictive.

### EXAMPLES

The invention will now be described with reference to specific examples, which should not be construed as in any way limiting.

### Example 1

### 1.1 Materials and Methods

### 1.1.1 Cell lines and patient samples

Human embryonic kidney (HEK293) (American Type Culture Collection, Manassas, VA, USA) and cells were routinely cultured in DMEM or RPMI supplemented with 10% FCS. All cells were incubated at 37°C in a humidified 5% CO₂ atmosphere.

Twenty-one formalin fixed paraffin embedded parathyroid tumour samples consisting of 11 parathyroid carcinomas and 10 parathyroid adenomas were obtained from Royal North Shore Hospital, Sydney, NSW, Australia according to protocols in place from the institution's Human Research Ethics committee.

### 1.1.2 Plasmid constructs

HRPT2 was PCR amplified using AccuPrime Pfx (Invitrogen Australia Pty Ltd, Vic:, Australia) from HEK293 cDNA using the primers:
5'-TAATCTCGAGCTATGGCGGACGTGCTTAGCGT-3' (SEQ ID NO: 21)
5'-TGCTCTCGAGTCTCCTTGAAGCACAAAGCAT-3' (SEQ ID NO: 22)
and the following PCR cycles: denaturation at 95°C for 2 min and then 35 cycles of 95°C for 1 min, annealing at 58°C for 1 min, extension at 68°C for 2 min followed by a final extension at 72°C for 10 min. PCR amplified HRPT2 was then cloned into the XhoI/HindIII sites of pEGFP-C1 (Clontech). Full length human HRPT2 (CDC73) was PCR amplified from pEGFP-C1-HRPT2 using primers 1 and 2, 3 and 4 or 5 and 6 (Table 1). CDC73 was cloned using the restriction sites present in each primer pair into the yeast expression vector pGBKT7 (Clontech, Clontech Laboratories Inc. Mountain View, CA, USA) to generate pGBKT7-CDC73; the mammalian expression vector pCMV-Myc (Clontech) to create pCMV-Myc-CDC73; and into the mammalian expression vector pCDNA4/TO (Invitrogen, Mount Waverly, VIC, Australia) to produce pCDNA4/TO-CDC73. Full length human RNF20 was PCR amplified using primers 7 and 8 **(Table 1)** from an adult human kidney cDNA library vector (pACT2) and cloned into the SalI and NotI restriction sites in frame with the HA-tag of the mammalian expression vector pCMV-HA to generate pCMV-HA-RNF20. CDC73. mutants were generated using the QuickChange II site directed mutagenesis kit (Stratagene, La Jolla, CA, USA) according to the manufacturer's instructions. Each mutation and the primers used for mutagenesis are listed in **Table 1.** All mutagenesis was verified by sequence analysis (Supamac, The University of Sydney and the Australian Genome Research Facility, Sydney, NSW, Australia).

**Table 1: Primers used to generate parafibromin mutants. Mutations are bolded or underlined.**

| Mutation | Primers used to generate parafibromin mutants |
|---|---|
| 308CGF | CTCAATGGTGAAGCGT**G**AACATCGGCAAGTATAG (SEQ ID NO: 1) |
| 308CGR | CTATACTTGCCGATGTT**C**ACGCTTCACCATTGAG (SEQ ID NO: 2) |
| 322delAF | GTCAACATCGGCAAGT_TAGACAGAAGCGCTCC (SEQ ID NO: 3) |
| 322delAR | GGAGCGCTTCTGTCTA_ACTTGCCGATGTTGAC (SEQ ID NO: 4) |
| 331TGAF | CGGCAAGTATAGACAGA**TGA**GCTCCCTTAGAAATAGGTC (SEQ ID NO: 5) |
| 331TGAR | GACCTATTTCTAAGGGAGC**TCA**TCTGTCTATACTTGCCG (SEQ ID NO: 6) |
| 341TGF | GACAGAAGCGCTCCCTGA**G**AAATAGGTCTTCAGCG (SEQ ID NO: 7) |
| 341TGR | CGCTGAAGACCTATTTCT**C**AGGGAGCGCTTCTGTC (SEQ ID NO: 8) |
| 700CTF | GAGTATGGAGGACA**T**GAACAACTATCTTAC (SEQ ID NO: 9) |
| 700CTR | GTAAGATAGTTGTTC**A**TGTCCTCCATACTC (SEQ ID NO: 10) |
| 1230DelCF | CAAAGAAGAAAAGA_CAGATGCAACCAGGGG (SEQ ID NO: 11) |
| 1230DelCR | CCCCTGGTTGCATCTG_TCTTTTCTTCTTTG (SEQ ID NO: 12) |
| 322ATGCF | TCAACATCGGCAAGT**GC**AGACAGAAGCGCTCC (SEQ ID NO: 13) |
| 322ATGCR | GGAGCGCTTCTGTCT**GC**ACTTGCCGATGTTGA (SEQ ID NO: 14) |
| 326ALAF | TCAACATCGGCAAGTATAGCC**GC**AAGCGCTCCCTTAGAAATA (SEQ ID NO: 15) |
| 326ALAR | TATTTCTAAGGGAGCGCTT**GC**G**G**CTATACTTGCCGATGTTGA (SEQ ID NO: 16) |
| 322ATGC2F | TCAACATCGGCAAGT**GC**AG**C**C**GC**AAGCGCTCC (SEQ ID NO: 17) |
| 322ATGC2R | GGAGCGCTT**GC**G**G**CT**GC**ACTTGCCGATGTTGA (SEQ ID NO: 18) |

| | Primers used to disrupt parafibromin siRNA binding |
|---|---|
| siRNAF | 5'-AAAGAAGAAACAAGGATGCCAGCGAGAAAATGAAAC-3' (SEQ ID NO: 19) |
| siRNAR | 5'-GTTTCATTTTCTCGCTGGCATCCTTGTTTCTTCTTT-3' (SEQ ID NO: 20) |

### 1.1.3 CDC73 yeast two hybrid screen

The Matchmaker 3 system (Clontech, place) was used to perform a high stringency yeast two-hybrid screen of full length CDC73. Reporter gene activation was not observed upon transformation of the yeast strain AH109 with pGBKT7-CDC73 suggesting that CDC73 alone did not induce Gal4 mediated transcription in this strain of yeast. pGBKT7-CDC73 and an adult human kidney cDNA library (Clontech) generated in the Gal4 activation domain yeast expression vector pACT2 were simultaneously transformed into AH109 cells and plated on high stringency dropout media (SD -Trp, -Leu, -His, -Ade and X-α-Gal). Plasmid DNA was isolated from resultant colonies and retransformed into the JM109 *E.coli* bacterial strain. Selection was performed on ampicillin to isolate clones containing the pACT2 vector only. DNA extracted from bacterial clones was sequenced using a T7 primer (Supamac, The University of Sydney, Australia).

### 1.1.4 Co-immunoprecipitation studies

For co-immunoprecipitation of endogenously expressed proteins, T175 culture dishes containing confluent monolayers of HEK293 cells were used. For co-immunoprecipitation of overexpressed CDC73 and RNF20, HEK293 cells were seeded (3x10⁵ cells/well) in 6 well culture dishes and 24h later co-transfected with 1 µg of pCMV-HA-RNF20 and pCMV-Myc-CDC73. 48h later cells were trypsinised, pelleted and resuspended in lysis buffer (300µl of 20mM Tris pH 7.5, 150 mM NaCl, 1mM EGTA, 1mM EDTA, 0.1% TX100, 10µg/ml leupeptin, 10µg/ml aprotinin, 1mM PMSF). Cell lysates were sonicated (10s), centrifuged (14,000g for 10min) and the supernatant used in co-immunoprecipitations. Anti-mouse or anti-rabbit IgG coated magnetic dynabeads (Invitrogen Australia Pty Ltd., Mulgrave, VIC, Australia) (50 µl/sample) were blocked in 1% BSA in PBS for 1h at 4°C. Beads were incubated in lysis buffer containing an anti-HA or anti-Myc antibody (Cell Signalling Technology, Beverly, MA, USA) or an IgG control (Pierce Biotechnology, Rockford, IL, USA) for 1.5h at 4°C. Beads were washed (3x1ml lysis buffer) and the supernatant from prepared cell lysates incubated with primary antibody coated beads for 1.5h at 4°C. Beads were washed a further six times (1ml lysis buffer, 500mM NaCl) for 10 min at 4°C. To remove immunoprecipitated material from beads, protein loading buffer (10µl of 6% w/v SDS, 40% w/v sucrose, 20mM Tris pH 6.8, 0.15% w/v bromophenol blue, 5% v/v β-mercaptoethanol) was added and incubated at 95°C for 5 min.

Extracts were removed from beads, separated on SDS-PAGE and analysed by western blots using HA and Myc-tag antibodies. Cell lysates containing 20 µg of protein were loaded onto 12% PAGE and electrophoresed at 25 A for 45 min (Bio-Rad Laboratories Pty Ltd, NSW, Australia). Proteins were then electrophoretically transferred to nitrocellulose (Amersham Biosciences Pty Ltd, NSW, Australia) for 1.5 h at 25 A. Nonspecific protein binding sites were then blocked by a 1 h incubation in blocking buffer (40 mM Tris (pH 7.4), 0.1% Tween-20, 5% skim milk). The nitrocellulose membrane was incubated with HA and/or Myc-tag antibodies (diluted 1/5000 in 40 mM Tris, 0.1% Tween-20, 5% skim milk) (Roche Diagnostics Australia Pty Ltd, NSW, Australia) for 1 h and subsequently rinsed in wash buffer (3 x5 min in 20 mM Tris (pH 7.4), 0.1% Tween-20, 150 mM NaCl). Incubation with an anti-mouse or anti-rabbit immunoglobulin HRP-conjugated secondary antibody (Amersham Biosciences Pty Ltd, NSW, Australia) (diluted 1/2500 in 40 mM Tris, 0.1% Tween-20, 5% skim milk) was then carried out for 1 h. The nitrocellulose was rinsed in wash buffer as above and developed using enhanced chemiluminescence (Pierce Biotechnology Inc., IL, USA) as per the manufacturer's instructions.

### 1.1.5 siRNA induced downregulation of CDC73 and subsequent analysis of monoubiquitinated H2B 7

HEK293 cells (3x10⁵ cells/well) were seeded into 6 well culture dishes and 24h later transfected with 100nM siRNA (target sequence - GAAGCGTCAACATCGGCAAGTA (SEQ ID NO: 23)) using 12µl HiPerfect (Qiagen Pty Ltd., Doncaster, VIC, Australia) according to the manufacturer's instructions. After 48h, cells were washed with cold PBS and lysed (200µl of 20 mM NaPO4, 0.5 M NaCl, 20mM Imidazole, 8 M urea, 0.5 % triton, 20 mM Tris pH 8, 0.5 mM DTT, 0.5 mM iodacetamide). Cells were removed from dishes by scraping and protein loading buffer added (100µl of 6% w/v SDS, 40% w/v sucrose, 20mM Tris pH 6.8, 0.15% w/v bromophenol blue). Extracts were sonicated (2x30s) and incubated at 95°C for 5min before being separated on SDS-PAGE and analysed by western blot using an anti-monoubiquitinated H2B (K120) antibody (Medimabs, Montreal, Canada).

### 1.1.6 Immunohistochemistry

Immunohistochemistry of monoubiquitinated H2B, total H2B and tri-Methyl-Histone 3 (lys4) was performed on 4 µM sections of formalin fixed paraffin embedded tissue on positively charged slides (Superfrost plus, Menzel-Glaser, Germany). A mouse monoclonal antibody raised against monoubiquitinated H2B (Medimabs Montreal Canada, Cat# MM-0029) was used at a dilution of 1 in 2000. Total H2B was used at a dilution of 1 in 500. A rabbit monoclonal antibody raised against tri-Methyl-Histone 3 (Lys4) (Cell Signalling Technology, MA, USA) was used at a dilution of 1 in 200. All slides were processed with an automated staining system - the Vision Biosystems BondmaX autostainer (Vision Biosystems, Mount Waverley, Victoria, Australia) according to the manufacturer's protocol and with the manufacturer's retrieval solutions. For monoubiquitinated H2B, heat induced epitope retrieval was performed at 97°C for 30 min in the manufacturer's acidic retrieval solution ER1 (VBS part no: AR9961). For triMethyl-Histone 3 (Lys4), heat induced epitope retrieval was performed at 97°C for 9 30min in the manufacturer's alkaline retrieval solution ER2 (VBS part no: AR9640). A biotin free detection system was employed (VBS part no: DS 9713).

### 1.1.7 Statistical analysis

Data analysis was performed using SPSS software 17.0 (SPSS Australasia Pty Ltd, Chatswood, NSW, Australia). Data are expressed as mean ± S.E.M from three independent Western blot experiments. Statistical significance for Western blot analysis was determined by one-way ANOVA. P<0.05 was considered statistically significant.

### 1.2 Results

In the experimental results discussed below and provided in the Figures, the human histone H2B sequence referred to is 125 amino acids in length. The skilled addressee will recognise that references to various residues in the sequence are equally applicable to corresponding residues in a human histone H2B sequence of different length. For example, a reference to "lysine 120" or "K120" is equivalent to "lysine 121" or "K121" in a human histone H2B sequence of 126 amino acids in length, such as one that commences with an initiating methionine residue.

### 1.2.1 The ring finger proteins RNF20 and RNF40 interact with CDC73

A high stringency yeast two-hybrid screen of an adult human kidney cDNA library using CDC73 as bait identified 53 unique putative binding partners of CDC73 from 371 yeast clones representing a diverse range of cellular functions (data not shown). Two of the most frequent putative interactors were the ring finger proteins RNF20 and RNF40. Co-transformation of CDC73 and RNF20 or RNF40 in a yeast two-hybrid assay confirmed this interaction (data not shown). RNF20 is the E3 ubiquitin ligase for histone H2B monoubiquitination at lysine 120 (K120) and RNF40 binds to RNF20. Given that RNF20 and RNF40 were identified with high frequency and play a significant role in transcription, they were selected for further analysis.

Firstly, an overexpression model was used to show interaction between CDC73 and RNF20 or RNF40 in mammalian cells. HA-tagged RNF20 or RNF40 and Myc-tagged CDC73 were transfected into HEK293 cells. Myc-tagged CDC73 was co-immunoprecipitated by an anti-HA antibody when overexpressed in the presence of HA-tagged RN20 or RNF40 **(****Figures 1A and 1B****).** In contrast, an IgG control antibody could not immunoprecipitate Myc-tagged CDC73 **(****Figures 1A and 1B****).** Furthermore, in the absence of expression of HA-RNF20 or HA-RNF40, neither an anti-HA nor IgG control antibody could immunoprecipitate Myc-CDC73 **(****Figures 1A and 1B**). These results were confirmed in a reverse immunoprecipitation in which an anti-Myc antibody was used to co-immunoprecipitate HA-tagged RNF20 or RNF40 (data not shown). Furthermore, in HEK293 cells that had not undergone transfection, endogenous RNF20 or RNF40 was co-immunoprecipitated using an antibody against CDC73 **(****Figure 1C****).** An IgG control antibody was unable to co-immunoprecipitate RNF20. Taken together, this data provides strong evidence that RNF20 and RNF40 are binding partners of CDC73.

### 1.2.2 Mapping the CDC73 RNF20 or RNF40 binding site

The yeast two-hybrid **assay** was again employed to determine the regions of CDC73 required for binding to RNF20 or RNF40. Several truncating mutations were introduced into pGBKT7-HRPT2 to allow expression of fragments of CDC73 fused to the Gal4 binding domain in yeast. Co-transformation of these constructs with full length RNF20 or RNF40 fused to the Gal4 activation domain and subsequent assessment of reporter gene activation revealed that CDC73 residues from 108-110 inclusive, were required for RNF20 binding **(****Figure 2A****)** while residues 98-100 inclusive, were required for RNF40 binding **(****Figure 2B****).** In order to ensure that no artefacts were introduced through potential misfolding of a truncated CDC73 protein, residues 108-110 or 98-100 were mutated to alanine in different combinations in full length CDC73. Co-transformation of these constructs with RNF20 or RNF40, followed by assessment of reporter gene activation demonstrated that the isoleucine at residue 108 of CDC73 was an absolute requirement for the association of CDC73 and RNF20 while residues 98, 99, 100 were required for association of CDC73 and RNF40 **(****Figure 2A** **and** **B****).** Alignment of the protein sequence of human CDC73 with that of multiple species revealed that the isoleucine at residue 108 is highly conserved. The only change observed at this position across these species was the presence of another neutral hydrophobic amino acid methionine in *C*. *elegans.* Residues 98, 99 and 100 were also highly conserved with only two changes observed at position 99 where asparagine had been replaced by the similar amino acids glutamine and threonine in *C*. *elegans* and *A. thaliana respectively* **(****Figure 2C****).**

### 1.2.3 RNF20 and CDC73 regulate endogenous levels of H2B monoubiquitination at K120

HEK293 cells were transiently transfected with siRNA directed against RNF20 or CDC73 for 48 hours after which the levels of RNF20 or CDC73 were reduced by 60% (mean 3 experiments). siRNA down-regulation of RNF20 reduced the levels of monoubiquitinated H2B (K120) by approximately 50%. siRNA down-regulation of CDC73 resulted in a similar reduction of monoubiquitinated H2B (K120) suggesting that CDC73 is required for monoubiquitination of H2B at K120 **(****Figures 3A and 3B****).** Despite leading to a decrease in the levels of monoubiquitinated histone H2B, down-regulation of RNF20 or CDC73 in this model did not affect levels of trimethylated H3-K4 **(****Figures 3A and 3B****).**

### 1.2.5 Loss or down-regulation of monoubiquitinated H2B-K120 in parathyroid carcinomas with loss of nuclear CDC73

Eleven parathyroid carcinomas exhibiting loss of nuclear CDC73 and 10 parathyroid adenomas that express nuclear CDC73 were examined by immunohistochemistry with antibodies against monoubiquitinated H2B (K120), total H2B and trimethylated H3-K4. The level of immunohistochemical staining was assessed for each sample and scored by a pathologist, 0 to 3, with 0 being low and 3 being the highest level of staining. Seventy percent of tumours that were positive for nuclear CDC73 exhibited high levels of monoubiquitinated H2B (scores 2 or 3), with the remaining 30% of tumours being scored 1. No CDC73 positive tumours were scored 0 for monoubiquitinated H2B. In contrast, all CDC73 negative tumours exhibited low levels of immunostaining for monoubiquitinated H2B (scores 0 or 1), with 57% of tumours being scored a 0. The level of total H2B in all tumours was consistently high, being scored 3 **(****Figures 4A** **and** **4C****).** Calculation of the mean immunohistochemical score for each tumour type revealed that CDC73 negative tumours expressed significantly less monoubiquitinated H2B compared to CDC73 positive tumours **(****Figure 4B****).** In contrast, no difference was observed in the levels of H3-K4 between CDC73 positive and negative tumours (data not shown).

### Example 2

### Materials and Methods

Commercial tissue arrays (US Biomax, MD, USA) were used to generate immunohistochemical data indicative of levels of histone 2B monoubiquitination in different tumour types including melanoma, breast and ovarian cancer, colorectal and lung cancer.

### Results

The results from immunohistochemical staining indicated that histone 2B monoubiquitination may be down-regulated, or absent, in a number of different tumour types including melanoma, breast and ovarian cancer, colorectal and lung cancer, while total histone 2B is expressed **(****Figure 5****).** It is apparent that monoubiquitinated histone 2B staining is lost in some tumour specimens, compared to total histone 2B which is retained.

Both monoubiquitinated histone 2B as well as total histone 2B staining was observed in normal tissue **(****Figure 6****).**

As indicated in **Figure 5** it would seem that breast tumours display decreased, or absent, histone 2B monoubiquitination. A proportion of breast tumours are also known to harbour mutations in the breast cancer 1 or 2 (*BRCA1* or *BRCA2*) genes. BRCA1 and BRCA2 function in the repair of double strand breaks *via* the homologous recombination pathway. *BRCA* mutations occur in a number of tumours, including breast, ovarian and pancreatic cancers.

Many laboratories are seeking to determine a marker of "BRCAness". A major reason for this is that tumours with down-regulation of BRCA appear to respond better to PARP (poly (ADP-ribose) polymerase) inhibitors. Therefore, an ability to better identify patients that will respond to PARP inhibitors has the potential to make a major difference to the clinical management of these patients.

Figure 6 provides indication that a breast cancer harbouring a *BRCA1* mutation has lost monoubiquitination of histone 2B, while retaining expression of total histone 2B. Of note, a mutant BRCA2 tumour did not show loss of histone 2B monoubiquitination (data not shown), indicating that loss of histone 2B monoubiquitination may have specific potential to identify *BRCA1*, but not *BRCA2* mutated tumours.

## Claims

1. A method for diagnosing cancer in a subject, the method comprising measuring histone 2B protein monoubiquitination in a first cell obtained from the subject, wherein decreased histone 2B protein monoubiquitination in said first cell compared to that of a second non-cancerous cell is diagnostic of cancer.

2. The method according to claim 1, wherein the second non-cancerous cell is obtained from the same subject.

3. The method according to claim 1 or claim 2, wherein the second non-cancerous cell is obtained from a different individual.

4. The method according to any one of claims 1 to 3, wherein the first and second cells are the same cell type.

5. The method according to any one of claims 1 to 4, wherein said cancer is any one or more of a parathyroid cancer, or cancer of the colon, breast, lung, prostate, ovary, brain, skin, pancreas, liver, oesophagus, thyroid gland, endometrium, pituitary gland, adrenal gland, breast, or prostate gland.

6. The method according to any one of claims 1 to 5, wherein said cancer is parathyroid cancer, breast cancer, colorectal cancer, lung cancer, melanoma, adrenal cancer, or ovarian cancer.

7. The method according to any one of claims 1 to 6, wherein the cancer is **characterised by** a mutation in the *BRCA1* gene.

8. The method according to any one of claims 1 to 7, wherein histone protein monoubiquitination in said first cell is decreased by at least 10% compared to histone protein monoubiquitination in said second non-cancerous cell.

9. The method according to any one of claims 1 to 8, wherein said measuring of histone protein monoubiquitination is performed using an antibody.

10. The method according to any one of claims 1 to 9, wherein said measuring of histone protein monoubiquitination is performed using any one or more of immunohistochemical staining, Western blotting and fluorescent activated cell sorting.

## Patentansprüche

1. Verfahren zur Diagnose von Krebs in einem Subjekt, wobei das Verfahren das Messen der Monoubiquitinierung des Histon-2B-Proteins in einer ersten, von dem Subjekt erhaltenen Zelle umfasst, wobei eine verminderte Monoubiquitinierung des Histon-2B-Proteins in der ersten Zelle im Vergleich zu der einer zweiten, nicht kanzerösen Zelle diagnostisch für Krebs ist.

2. Verfahren nach Anspruch 1, bei dem man die zweite, nicht kanzeröse Zelle von dem gleichen Subjekt erhält.

3. Verfahren nach Anspruch 1 oder 2, bei dem man die zweite, nicht kanzeröse Zelle von einem anderen Individuum erhält.

4. Verfahren nach einem der Ansprüche 1 bis 3, wobei die erste Zelle und die zweite Zelle vom gleichen Zelltyp sind.

5. Verfahren nach einem der Ansprüche 1 bis 4, wobei es sich bei der Krebserkrankung um eine oder mehrere ausgewählt aus Nebenschilddrüsenkrebs oder Krebs des Kolons, der Brust, der Lunge, der Prostata, der Eierstöcke, des Hirns, der Haut, der Bauchspeicheldrüse, der Leber, der Speiseröhre, der Schilddrüse, des Endometriums, der Hirnanhangsdrüse, der Nebenniere, der Brust oder der Prostatadrüse handelt.

6. Verfahren nach einem der Ansprüche 1 bis 5, wobei es sich bei der Krebserkrankung um Nebenschilddrüsenkrebs, Brustkrebs, Kolorektalkrebs, Lungenkrebs, Melanom, Nebennierenkrebs oder Eierstockkrebs handelt.

7. Verfahren nach einem der Ansprüche 1 bis 6, wobei die Krebserkrankung durch eine Mutation im *BRCA1*-Gen charakterisiert ist.

8. Verfahren nach einem der Ansprüche 1 bis 7, wobei die Monoubiquitinierung des Histonproteins in der ersten Zelle im Vergleich zur Monoubiquitinierung des Histonproteins in der zweiten, nicht kanzerösen Zelle um mindestens 10% vermindert ist.

9. Verfahren nach einem der Ansprüche 1 bis 8, bei dem das Messen der Monoubiquitinierung des Histonproteins mit einem Antikörper erfolgt.

10. Verfahren nach einem der Ansprüche 1 bis 9, wobei das Messen der Monoubiquitinierung des Histonproteins unter Verwendung eines oder mehrerer von immunhistochemischer Anfärbung, Western-Blotting und Fluorescence-activated Cell Sorting erfolgt.

## Revendications

1. Procédé de diagnostic du cancer chez un sujet, le procédé comprenant la mesure de la mono-ubiquitination de la protéine histone 2B dans une première cellule obtenue du sujet, dans lequel la diminution de la mono-ubiquitination de la protéine histone 2B dans ladite première cellule comparativement à celle d'une seconde cellule non cancéreuse est un diagnostic de cancer.

2. Procédé selon la revendication 1, dans lequel la seconde cellule non cancéreuse est obtenue à partir du même sujet.

3. Procédé selon la revendication 1 ou la revendication 2, dans lequel la seconde cellule non cancéreuse est obtenue à partir d'un individu différent.

4. Procédé selon l'une quelconque des revendications 1 à 3, dans lequel les première et seconde cellules sont du même type cellulaire.

5. Procédé selon l'une quelconque des revendications 1 à 4, dans lequel ledit cancer est l'un quelconque ou plusieurs d'un cancer de la parathyroïde, ou un cancer du côlon, du sein, du poumon, de la prostate, de l'ovaire, du cerveau, de la peau, du pancréas, du foie, de l'oesophage, de la glande de la thyroïde, de l'endomètre, de la glande de l'hypophyse, de la glande surrénale, du sein, ou de la glande de la prostate.

6. Procédé selon l'une quelconque des revendications 1 à 5, dans lequel ledit cancer est un cancer de la parathyroïde, un cancer du sein, un cancer colorectal, un cancer du poumon, un mélanome, un cancer de la surrénale, ou un cancer ovarien.

7. Procédé selon l'une quelconque des revendications 1 à 6, dans lequel le cancer est **caractérisé par** une mutation dans le gène *BRCA1.*

8. Procédé selon l'une quelconque des revendications 1 à 7, dans lequel la monoubiquitination de la protéine histone dans ladite première cellule est diminuée d'au moins 10 % comparativement à la mono-ubiquitination de la protéine histone dans ladite seconde cellule non cancéreuse.

9. Procédé selon l'une quelconque des revendications 1 à 8, dans lequel ladite mesure de la mono-ubiquitination de la protéine histone est effectuée en utilisant un anticorps.

10. Procédé selon l'une quelconque des revendications 1 à 9, dans lequel ladite mesure de la mono-ubiquitination de la protéine histone est effectuée en utilisant l'un(e) quelconque ou plusieurs d'une coloration immunohistochimique, une analyse Western blot et un tri de cellules activées fluorescentes.


### SEQUENCE LISTING

<110> Northern Sydney Local Health District
<120> Cancer Biomarkers
<130> 965069C
<160> 44
<170> PatentIn version 3.2
<210> 1
   <211> 34
   <212> DNA
   <213> Homo sapiens
<400> 1
   ctcaatggtg aagcgtgaac atcggcaagt atag 34
<210> 2
   <211> 34
   <212> DNA
   <213> Homo sapiens
<400> 2
   ctatacttgc cgatgttcac gcttcaccat tgag 34
<210> 3
   <211> 32
   <212> DNA
   <213> Homo sapiens
<400> 3
   gtcaacatcg gcaagttaga cagaagcgct cc 32
<210> 4
   <211> 32
   <212> DNA
   <213> Homo sapiens
<400> 4
   ggagcgcttc tgtctaactt gccgatgttg ac 32
<210> 5
   <211> 39
   <212> DNA
   <213> Homo sapiens
<400> 5
   cggcaagtat agacagatga gctcccttag aaataggtc 39
<210> 6
   <211> 39
   <212> DNA
   <213> Homo sapiens
<400> 6
   gacctatttc taagggagct catctgtcta tacttgccg 39
<210> 7
   <211> 35
   <212> DNA
   <213> Homo sapiens
<400> 7
   gacagaagcg ctccctgaga aataggtctt cagcg 35
<210> 8
   <211> 35
   <212> DNA
   <213> Homo sapiens
<400> 8
   cgctgaagac ctatttctca gggagcgctt ctgtc 35
<210> 9
   <211> 30
   <212> DNA
   <213> Homo sapiens
<400> 9
   gagtatggag gacatgaaca actatcttac 30
<210> 10
   <211> 30
   <212> DNA
   <213> Homo sapiens
<400> 10
   gtaagatagt tgttcatgtc ctccatactc 30
<210> 11
   <211> 30
   <212> DNA
   <213> Homo sapiens
<400> 11
   caaagaagaa aagacagatg caaccagggg 30
<210> 12
   <211> 30
   <212> DNA
   <213> Homo sapiens
<400> 12
   cccctggttg catctgtctt ttcttctttg 30
<210> 13
   <211> 32
   <212> DNA
   <213> Homo sapiens
<400> 13
   tcaacatcgg caagtgcaga cagaagcgct cc 32
<210> 14
   <211> 32
   <212> DNA
   <213> Homo sapiens
<400> 14
   ggagcgcttc tgtctgcact tgccgatgtt ga 32
<210> 15
   <211> 42
   <212> DNA
   <213> Homo sapiens
<400> 15
   tcaacatcgg caagtatagc cgcaagcgct cccttagaaa ta 42
<210> 16
   <211> 42
   <212> DNA
   <213> Homo sapiens
<400> 16
   tatttctaag ggagcgcttg cggctatact tgccgatgtt ga 42
<210> 17
   <211> 32
   <212> DNA
   <213> Homo sapiens
<400> 17
   tcaacatcgg caagtgcagc cgcaagcgct cc 32
<210> 18
   <211> 32
   <212> DNA
   <213> Homo sapiens
<400> 18
   ggagcgcttg cggctgcact tgccgatgtt ga 32
<210> 19
   <211> 36
   <212> DNA
   <213> Homo sapiens
<400> 19
   aaagaagaaa caaggatgcc agcgagaaaa tgaaac 36
<210> 20
   <211> 36
   <212> DNA
   <213> Homo sapiens
<400> 20
   gtttcatttt ctcgctggca tccttgtttc ttcttt 36
<210> 21
   <211> 32
   <212> DNA
   <213> Homo sapiens
<400> 21
   taatctcgag ctatggcgga cgtgcttagc gt 32
<210> 22
   <211> 31
   <212> DNA
   <213> Homo sapiens
<400> 22
   tgctctcgag tctccttgaa gcacaaagca t 31
<210> 23
   <211> 22
   <212> DNA
   <213> Homo sapiens
<400> 23
   gaagcgtcaa catcggcaag ta 22
<210> 24
   <211> 9
   <212> PRT
   <213> Homo sapiens
<400> 24
   Ser Ala Ser Ile Asp Arg Ser Ala Pro
   1 5
<210> 25
   <211> 9
   <212> PRT
   <213> Homo sapiens
<400> 25
   Ser Ala Ser Ala Asp Arg Ser Ala Pro
   1 5
<210> 26
   <211> 9
   <212> PRT
   <213> Homo sapiens
<400> 26
   Ser Ala Ser Ile Ala Ala Ser Ala Pro
   1 5
<210> 27
   <211> 9
   <212> PRT
   <213> Homo sapiens
<400> 27
   Ser Ala Ser Ala Ala Ala Ser Ala Pro
   1 5
<210> 28
   <211> 8
   <212> PRT
   <213> Homo sapiens
<400> 28
   Gly Tyr Leu Asn Gly Glu Ala Ser
   1 5
<210> 29
   <211> 8
   <212> PRT
   <213> Homo sapiens
<400> 29
   Gly Tyr Leu Ala Gly Glu Ala Ser
   1 5
<210> 30
   <211> 8
   <212> PRT
   <213> Homo sapiens
<400> 30
   Gly Tyr Leu Asn Ala Glu Ala Ser
   1 5
<210> 31
   <211> 8
   <212> PRT
   <213> Homo sapiens
<400> 31
   Gly Tyr Leu Ala Ala Glu Ala Ser
   1 5
<210> 32
   <211> 8
   <212> PRT
   <213> Homo sapiens
<400> 32
   Gly Tyr Ala Ala Ala Glu Ala Ser
   1 5
<210> 33
   <211> 27
   <212> PRT
   <213> Homo sapiens
<400> 33
   Gly Tyr Leu Asn Gly Glu Ala Ser Thr Ser Ala Ser Ile Asp Arg Ser
   1 5 10 15
   Ala Pro Leu Glu Ile Gly Leu Gln Arg Ser Thr
   20 25
<210> 34
   <211> 27
   <212> PRT
   <213> Pan troglodytes
<400> 34
   Gly Tyr Leu Asn Gly Glu Ala Ser Thr Ser Ala Ser Ile Asp Arg Ser
   1 5 10 15
   Ala Pro Leu Glu Ile Gly Leu Gln Arg Ser Thr
   20 25
<210> 35
   <211> 27
   <212> PRT
   <213> Canis lupus
<400> 35
   Gly Tyr Leu Asn Gly Glu Ala Ser Thr Ser Ala Ser Ile Asp Arg Ser
   1               5                   10                  15
   Ala Pro Leu Glu Ile Gly Leu Gln Arg Ser Thr
               20                  25
<210> 36
   <211> 27
   <212> PRT
   <213> Bos taurus
<400> 36
   Gly Tyr Leu Asn Gly Glu Ala Ser Thr Ser Ala Ser Ile Asp Arg Ser
   1               5                   10                  15
   Ala Pro Leu Glu Ile Gly Leu Gln Arg Ser Thr
               20                  25
<210> 37
   <211> 27
   <212> PRT
   <213> Mus musculus
<400> 37
   Gly Tyr Leu Asn Gly Glu Ala Ser Thr Ser Ala Ser Ile Asp Arg Ser
   1               5                   10                  15
   Ala Pro Leu Glu Ile Gly Leu Gln Arg Ser Thr
               20                  25
<210> 38
   <211> 27
   <212> PRT
   <213> Rattus norvegicus
<400> 38
   Gly Tyr Leu Asn Gly Glu Ala Ser Thr Ser Ala Ser Ile Asp Arg Ser
   1               5                   10                  15
   Ala Pro Leu Glu Ile Gly Leu Gln Arg Ser Thr
               20                  25
<210> 39
   <211> 27
   <212> PRT
   <213> Gallus gallus
<400> 39
   Ala Tyr Leu Asn Gly Glu Thr Ser Thr Ser Ser Ser Ile Asp Arg Ser
   1               5                   10                  15
   Ala Pro Leu Glu Ile Gly Leu Gln Arg Ser Thr
               20                  25
<210> 40
   <211> 27
   <212> PRT
   <213> Danio rerio
<400> 40
   Ser Tyr Leu Asn Gly Glu Ser Ser Ser Ser Thr Ser Ile Asp Arg Ser
   1               5                   10                  15
   Ala Pro Ile Glu Ile Gly Leu Gln Arg Pro Thr
               20                  25
<210> 41
   <211> 23
   <212> PRT
   <213> Drosophila melanogaster
<400> 41
   Ala Tyr Leu Asn Gly Glu Thr Pro Thr Cys Ala Ser Ile Asp Lys Ser
   1               5                   10                  15
   Ala Pro Leu Glu Ile Pro Thr
               20
<210> 42
   <211> 23
   <212> PRT
   <213> Anopheles gambiae
<400> 42
   Gln Tyr Leu Asn Gly Glu Thr Asn Thr Cys Ala Ser Ile Asp Lys Ser
   1               5                   10                  15
   Ala Pro Leu Glu Ile Pro Thr
               20
<210> 43
   <211> 22
   <212> PRT
   <213> Caenorhabditis elegans
<400> 43
   Glu Tyr Gln Gly Asp Arg Thr Asp Phe Pro Ala Leu Met Asn Gln Val
   1               5                   10                  15
   Asn Pro Leu Ser Leu Arg
               20
<210> 44
   <211> 23
   <212> PRT
   <213> Arabidopsis thaliana
<400> 44
   Asp Tyr Leu Thr Gly Arg Val Ala Ser Ser Asp Ser Ile Asp Phe Leu
   1               5                   10                  15
   Leu Leu Gln Gln Gln Asn Ala
               20